(19) Europäisches Patentamt
European Patent Office
Office européen des brevets

(11) **EP 4 191 606 A1**

(12) **EUROPEAN PATENT APPLICATION**
published in accordance with Art. 153(4) EPC

(43) Date of publication:
**07.06.2023 Bulletin 2023/23**

(21) Application number: **21851465.1**

(22) Date of filing: **10.06.2021**

(51) International Patent Classification (IPC):
**G16H 40/60** (2018.01)    **A61B 34/00** (2016.01)

(52) Cooperative Patent Classification (CPC):
**A61B 34/00; G16H 40/60**

(86) International application number:
**PCT/JP2021/022041**

(87) International publication number:
**WO 2022/024559 (03.02.2022 Gazette 2022/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.07.2020 JP 2020131216**

(71) Applicant: **Sony Group Corporation
Minato-Ku, Tokyo, 108-0075 (JP)**

(72) Inventors:
• **SUZUKI, Kenji
Tokyo 108-0075 (JP)**
• **KURODA, Yohei
Tokyo 108-0075 (JP)**
• **NAGAO, Daisuke
Tokyo 108-0075 (JP)**
• **MATSUURA, Kana
Tokyo 108-0075 (JP)**

(74) Representative: **2SPL Patentanwälte PartG mbB
Landaubogen 3
81373 München (DE)**

(54) **MEDICAL ASSISTANCE SYSTEM, MEDICAL ASSISTANCE METHOD, AND COMPUTER PROGRAM**

(57)    Provided is a medical support system that supports medical practice by a doctor.

The medical support system includes: a control unit; a recognition unit that recognizes an operative field environment; and a machine learning model that estimates an operation performed by the medical support system on the basis of a recognition result of the recognition unit. The control unit outputs determination basis information regarding the operation estimated by the machine learning model to an information presentation unit. The control unit further includes a calculation unit that calculates reliability regarding an estimation result of the machine learning model, and outputs the reliability to the information presentation unit.

*FIG. 7*

**Description**

TECHNICAL FIELD

The technology disclosed in the present specification (hereinafter, "the present disclosure") relates to a medical support system, a medical support method, and a computer program that support medical practice by a doctor.

BACKGROUND ART

With the evolution of deep learning, high-precision inference beyond humans has been realized. Deep learning is also very promising in the medical field. For example, there has been proposed a medical information processing device including: an acquisition unit that acquires medical information; a learning unit that performs learning for a function in the medical information processing device using the medical information; an evaluation data holding unit that holds evaluation data for evaluating a learning result by the learning unit, the evaluation data having a known correct answer by execution of the function; an evaluation unit that evaluates the learning result acquired by learning on the basis of the evaluation data; and a reception unit that receives an instruction for applying the learning result of the learning unit to the function, in which the user determines whether or not the learning result is applied on the basis of a verification result of validity of learning (see Patent Document 1).

CITATION LIST

PATENT DOCUMENT

Patent Document 1: Japanese Patent Application Laid-Open No. 2020-27507

NON PATENT DOCUMENT

Non Patent Document 1: Alex Kendall, Yarin Gal, "What Uncertainties Do We Need in Bayesian Deep Learning for Computer Vison", NIPS 2017
Non Patent Document 2: Yarin Gal, Zoubin Ghahramani, "Dropout as Bayesian Approximation: Representing Model Uncertainty in Deep Learning", ICML 2016

SUMMARY OF THE INVENTION

PROBLEMS TO BE SOLVED BY THE INVENTION

An object of the present disclosure is to provide a medical support system, a medical support method, and a computer program that control an operation of an arm that supports a medical instrument such as an endoscope, for example, on the basis of an estimation result by deep learning.

SOLUTIONS TO PROBLEMS

A first aspect of the present disclosure is a medical support system including:

a control unit;
a recognition unit that recognizes an operative field environment; and
a machine learning model that estimates an operation performed by the medical support system on the basis of a recognition result of the recognition unit,
in which the control unit outputs determination basis information regarding the operation estimated by the machine learning model to an information presentation unit.

The control unit further includes a calculation unit that calculates reliability regarding an estimation result of the machine learning model, and outputs the reliability to the information presentation unit. The calculation unit calculates the reliability using Bayesian deep learning.
The machine learning model estimates a target command for an arm supporting a medical instrument. Then, the control unit outputs determination basis information regarding the target command estimated by the machine learning model to an information presentation unit. The control unit outputs information regarding a gaze region observed at the time of estimating the target command and/or a recognized target portion. The control unit outputs a heat map image indicating a gaze region observed at the time of estimating the target command and/or a recognized target portion. The control unit outputs the heat map image generated on the basis of the Grad-Cam algorithm.

Furthermore, a second aspect of the present disclosure is a medical support method in a medical support system, the medical support method including:

a recognition step of recognizing an operative field environment;
an estimation step of estimating, by a machine learning model, an operation performed by the medical support system on the basis of a recognition result in the recognition step; and
a step of outputting determination basis information regarding the operation estimated by the machine learning model to an information presentation unit.

Furthermore, a third aspect of the present disclosure is a computer program described in a computer readable format to execute processing of medical support in a medical support system on a computer, the computer program causing the computer to function as:

a recognition unit that recognizes an operative field environment;
an estimation unit that estimates, by a machine learning model, an operation performed by the medical support system on the basis of a recognition result in the recognition step; and
an output unit that outputs determination basis information regarding the operation estimated by the machine learning model to an information presentation unit.

The computer program according to the third aspect of the present disclosure defines a computer program described in a computer readable format so as to implement predetermined processing on a computer. In other words, by installing the computer program according to the third aspect of the present disclosure in a computer, a cooperative action is exerted on the computer, and it is possible to obtain a similar action and effect to those of the medical support system according to the first aspect of the present disclosure.

EFFECTS OF THE INVENTION

According to the present disclosure, for example, it is possible to provide an operation of an arm that supports a medical instrument that presents determination basis information and reliability of a machine learning model that estimates an operation of the arm that supports a medical instrument such as an endoscope.

Note that the effects described in the present specification are merely examples, and the effects brought by the present disclosure are not limited thereto. Furthermore, the present disclosure may further provide additional effects in addition to the effects described above.

Still other objects, features, and advantages of the present disclosure will become apparent from a more detailed description based on embodiments to be described later and the accompanying drawings.

BRIEF DESCRIPTION OF DRAWINGS

Fig. 1 is a diagram illustrating a configuration example of an endoscopic surgery system 100 to which a medical robot device can be applied.
Fig. 2 is a diagram illustrating a configuration example of a control system 200 that controls an operation of a medical robot device 120.
Fig. 3 is a diagram illustrating a functional configuration example of a camera head 112.
Fig. 4 is a diagram illustrating a functional configuration example of the medical robot device 120.
Fig. 5 is a diagram illustrating a configuration example of the control system 200 using a neural network model.
Fig. 6 is a diagram illustrating another configuration example of the control system 200 using a neural network model.
Fig. 7 is a diagram illustrating a configuration example of a control system 700 that presents a determination basis of motion prediction.
Fig. 8 is a diagram illustrating an example of a monitor image of an operative field.
Fig. 9 is a diagram illustrating a heat map image presented with respect to the monitor image illustrated in Fig. 8.
Fig. 10 is a diagram illustrating an example of an image for presenting target command-related information.
Fig. 11 is a diagram illustrating an example of an image for presenting explanation of reliability.

Fig. 12 is a diagram illustrating another example of an image for presenting explanation of reliability.

Fig. 13 is a flowchart illustrating a processing procedure for presenting a determination basis of motion prediction in the control system 700.

Fig. 14 is a diagram illustrating a configuration example of a control system 1400 that presents a determination basis of motion prediction.

Fig. 15 is a flowchart illustrating a processing procedure for presenting a determination basis of motion prediction in the control system 1400.

Fig. 16 is a diagram illustrating a display form in which an operative field image by an endoscope 110 and a presentation image of a determination basis are simultaneously displayed.

Fig. 17 is an enlarged view of a heat map image 1611 in Fig. 16.

Fig. 18 is an enlarged view of a heat map image 1612 in Fig. 16.

Fig. 19 is a diagram illustrating a procedure in a case where surgery is performed using an endoscopic surgery system 1000.

Fig. 20 is a flowchart illustrating an operation procedure in the endoscopic surgery system 1000 at the time of surgery.

Fig. 21 is a diagram illustrating a procedure for performing relearning of a learner.

Fig. 22 is a flowchart illustrating an operation procedure for performing relearning of a learner.

Fig. 23 is a diagram illustrating a procedure of performing autonomous learning of a learner.

Fig. 24 is a flowchart illustrating an operation procedure for performing autonomous learning of a learner.

## MODE FOR CARRYING OUT THE INVENTION

Hereinafter, the technology according to the present disclosure will be described in the following order with reference to the drawings.

A. Overview
B. Configuration of Endoscopic Surgery System
C. Control System of Medical Robot Device
D. Control System Using Neural Network Model
E. Control System for Presenting Determination Basis
F. Reflection of Determination of Doctor
G. Relearning of Learner
H. Autonomous Learning of Learner

A. Overview

For example, in endoscopic surgery, adjustment of a position, an angle of view, and the like of an endoscope influences a surgery performance, but a control technique is not constant for each operator (scopist). By introducing operation control of a robot based on inference by deep learning into a medical robot device that supports an endoscope, it is expected to reduce costs such as labor costs of an operator and to improve accuracy and safety of a control technology of the endoscope. Meanwhile, when deep learning is used, it is necessary to clarify the validity of the determination basis. This is because it is possible to confirm validity of the determination and no difference from the determination of the doctor by clarifying the basis of the determination of the medical robot device.

In recent years, researches on artificial intelligence that can be explained have become active, and algorithms that visualize the basis of determination and algorithms that reveal data uncertainty have been proposed. According to the present disclosure, when a medical robot device is controlled on the basis of inference by deep learning, not only the result of the inference by the deep learning but also the basis of the determination is presented. Furthermore, according to the present disclosure, uncertainty of control of a medical robot device based on inference by deep learning is also presented. It is possible to clarify whether determination by deep learning cannot be made due to noise.

Regarding the former case of the clarification of the determination basis, an image region regarding the determination basis can be displayed on a captured image of an endoscope in a heat map by Grad-Cam, which is one of explainable AI (XAI) algorithms, for example. The deep learned neural network model calculates target command value related information to the medical robot device using an input image (or a captured image of the endoscope) to the medical robot device, motion information (including the self-position of the camera) of the medical robot device, operation information, and sensor information as input data. The Grad-Cam can explicitly indicate which portion of the input image the neural network model has focused on to output the target command-related information by, for example, a method of displaying a heat map of an image region that is a basis. Here, the determination basis includes, for example, analysis information necessary for improving a data set or a machine learning model used for learning the machine learning model and debugging performance. Furthermore, the determination basis may be a prediction, a score, or the like indicating how much a certain factor influences the final result. The determination basis is only required to be, for example, information for analyzing a cause for a certain result.

The latter case of the uncertainty in deep learning can be mainly divided into uncertainty due to noise and uncertainty due to lack of data. For example, Bayesian deep learning can evaluate the uncertainty of the estimation result by the neural network, that is, how correctly the medical robot device is likely to move and process on the basis of the target command-related information output by the neural network model.

Therefore, by presenting the determination basis of the medical robot device and its uncertainty or reliability, the doctor can perform the endoscopic surgery while confirming whether the medical robot device is controlled without a difference from his/her own determination.

B. Configuration of Endoscopic Surgery System

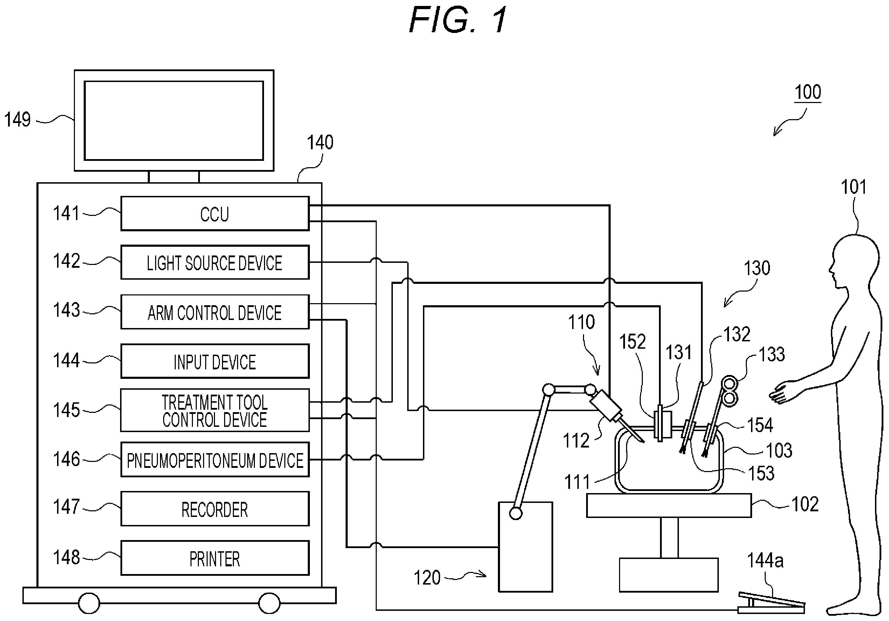

Fig. 1 schematically illustrates a configuration example of an endoscopic surgery system 100 to which a medical robot device according to the present disclosure can be applied. Fig. 1 illustrates a state in which a surgeon (doctor) 101 is performing surgery on a patient 103 on a surgical bed 102 using an endoscopic surgery system 100. The endoscopic surgery system 100 includes an endoscope 110, a medical robot device 120 that supports the endoscope 110, a medical instrument group 130 other than the endoscope 110, and a cart 140 on which various devices for endoscopic surgery are mounted.

The medical robot device 120 is basically a multilink structure in which a plurality of links is connected by joint shafts. The medical robot device 120 supports the endoscope 110 at the distal end portion. The medical robot device 120 has a freedom degree structure capable of controlling the posture of the endoscope 110, for example, at four degrees of freedom or more, securing a sufficient operation range of the endoscope during surgery, coping with various manual works, and avoiding interference with the surgeon 101.

Examples of the device mounted on the cart 140 include a camera control unit (CCU) 141, a light source device 142, a robot arm control device 143, an input device 144, a treatment tool control device 145, an air film device 146, a recorder 147, a printer 148, a display device 149, and the like. However, the types of devices mounted on the cart 140 can be appropriately changed according to the types of medical instruments used for endoscopic surgery.

In endoscopic surgery, instead of cutting and opening the abdominal wall of the patient 103, a lens barrel of the endoscope 110 and other medical instruments 131, 132, ... are inserted into a body cavity of the patient 103 via a plurality of trocars 151, 152, ... punctured into the abdominal wall. The medical instruments 131, 132, ... are, for example, forceps, pneumoperitoneum tubes, energy treatment tools, tweezers, retractors, and the like, but are illustrated in a simplified manner in Fig. 1.

An image of the surgical site in the body cavity of the patient 103 captured by the endoscope 110 is displayed on the display device 149. The surgeon 101 performs treatment such as resection of the surgical site using the medical instruments 131, 132, ... while viewing the image of the surgical site displayed on the display device 149 in real time. Furthermore, some of the medical instruments 131, 132, ... (for example, forceps, a pneumoperitoneum tube, and an energy treatment tool) may be supported by an assistant (not illustrated) instead of the surgeon.

The endoscope 110 includes a lens barrel 111 inserted into the body cavity of the patient 103 at the distal end, and a camera head 112 connected to the proximal end of the lens barrel 111. The lens barrel 111 is assumed to be a rigid mirror including a lens barrel having a configuration, but may be a flexible mirror including a flexible lens barrel. An optical system and an imaging element (both not illustrated) are disposed in the camera head 112. Reflected light (observation light) from an observation target such as a surgical site is imaged on the imaging element by the optical system. The imaging element photoelectrically converts the observation light, generates an image signal corresponding to the observation image, and transmits the image signal to the CCU 141. Note that the camera head 112 has a function of driving the optical system to adjust magnification and a focal length. Furthermore, a plurality of imaging elements may be disposed in the camera head 112 for stereoscopic viewing (3D display). In this case, a plurality of relay optical systems for guiding the observation light to the plurality of imaging elements is disposed inside the lens barrel 111.

The CCU 141 includes a central processing unit (CPU), a graphics processing unit (GPU), and the like, performs control of the camera head 112 of the endoscope 110, processing of a captured image of the abdominal cavity captured by the endoscope 110, and signal processing on a pixel signal acquired by controlling the camera head 112, and controls screen display of the captured image of the endoscope 110 by the display device 149. Specifically, the CCU 141 performs development processing such as demosaic on the image signal received from the camera head 112 and various image processing for displaying an image, and outputs the image signal to the display device 141. In the present embodiment, the CCU 141 includes an image recognizer including a neural network model learned by deep learning, recognizes an object, an environment, and the like in a field of view of the endoscope 110 from a captured image subjected to image processing, and outputs recognition information. Furthermore, the CCU 141 transmits a control signal related to adjustment of the magnification and the focal length and an imaging condition to the camera head 112.

The display device 149 displays a captured image of the endoscope 110 on which image processing has been performed by the CCU 141. It is preferable to use the display device 149 having an appropriate resolution or screen size according to the application. For example, in a case where the endoscope 110 supports high-resolution imaging such as 4K (the number of horizontal pixels 3840 × the number of vertical pixels 2160) or 8K (the number of horizontal pixels 7680 × the number of vertical pixels 4320), or supports 3D display, it is preferable to use a display device capable of high-resolution or 3D display as the display device 149. By using, for example, a display device having a screen size of 55 inches or more as the display device 149 compatible with high resolution of 4K or 8K, it is possible to give a further immersive feeling to an observer such as the surgeon 101.

The light source device 142 includes, for example, a light source such as a light emitting diode (LED) or a laser, and supplies illumination light to the endoscope 110 when imaging a surgical site.

The robot arm control device 143 includes, for example, a processor such as a CPU, a local memory thereof, and the like, and controls operations of the camera head 112 and the medical robot device 120. The robot arm control device 143 controls driving of the robot arm of the medical robot device 120 according to a predetermined control method such as position control and force control, for example. The medical robot device 120 has a multilink structure in which a plurality of links is connected by joint shafts and the endoscope 110 is mounted on a distal end portion, and at least some of the joint shafts is an active shaft driven by an actuator. The robot arm control device 143 supplies a drive signal to each joint driving actuator.

In the present embodiment, the robot arm control device 143 includes a motion predictor including a neural network model learned by deep learning, and predicts and outputs a target command value for the camera head 112 and the medical robot device 120 to be controlled on the basis of the recognition information recognized by the image recognizer (described above). The target command value is a value indicating a control amount with respect to a control target, and specifically includes information regarding camerawork of the endoscope 110 (camera target position, posture, speed, acceleration, gaze point, and line-of-sight vector (target object position, distance, vector posture)), a predicted image captured by the endoscope 110 (including an electronic cut-out position of the captured image), and a predicted operation of the robot arm of the medical robot device 120 (target position, posture, speed, acceleration, operation force, and the like, of the instruments supported by the robot arm). Note that, instead of individually arranging the neural network model of the image recognizer in the CCU 141 and the neural network model of the motion predictor in the robot arm control device 143, image recognition and motion prediction may be integrated and configured as an End to End (E2E) neural network model. Furthermore, in the present disclosure, a determination basis, uncertainty, or reliability by the neural network model is presented, but details will be described later.

The input device 144 is an input interface for the endoscopic surgery system 100. A user (for example, a surgeon, a doctor, an assistant, and the like) can input various types of information and instructions to the endoscope system 100 via the input device 144. For example, the user inputs various types of information regarding surgery, such as physical information of a patient and information related to surgery, via the input device 144. Furthermore, the user (for example, a surgeon, a doctor, an assistant, and the like) inputs an instruction to drive the medical robot device 120, setting of imaging conditions (type of irradiation light, magnification, focal length, and the like) by the endoscope 110, a drive instruction of the energy treatment tool, and the like via the input device 144. In the present disclosure, the determination basis, uncertainty, or reliability of the neural network model for estimating the operation of the robot arm of the medical robot device 120 is presented, but the user can input an instruction to the medical robot device 120 via the input device 144 according to the presented content.

The type of the input device 144 is not limited. The input device 144 may be, for example, a mouse, a keyboard, a touch panel, a switch, a lever (none of which are illustrated), a foot switch 144a, or the like. The touch panel is superimposed on a screen of the display device 149, for example, and the user can perform an input operation on a captured image of the endoscope 110 displayed on the screen, for example. Furthermore, as the input device 144, a head mounted display or various types of wearable devices may be used to input information according to the user's line-of-sight or gesture. Furthermore, the input device 144 may include a master device of the medical robot device 120. Furthermore, the input device 144 may include a microphone that collects the voice of the user, and may input a voice command from the user. By using a device capable of inputting information in a non-contact manner for the input device 144, a user in a clean area in an operating room can operate a device arranged in an unclean area in a non-contact manner, and the user can input information without releasing his/her hand from the medical instruments 131, 132, ....

A treatment tool control device 145 controls driving of an energy treatment tool for cauterization and incision of tissue, sealing of a blood vessel, and the like. For the purpose of securing a field of view by the endoscope 110 and securing a working space of the surgeon, the air film device 146 sends gas into the body cavity of the patient 103 through the undulating tube to inflate the body cavity. The recorder 147 includes, for example, a large-capacity recording device such as a solid state drive (SSD), a hard disc drive (HDD), and the like, and is used for recording various types of information regarding surgery. The printer 148 is a device that prints data such as characters, images, and figures on paper, and is used to print information regarding surgery. The treatment tool control device 145 and the pneumoperitoneum device 146 operate, for example, on the basis of an instruction from the surgeon 101 or an assistant via the input device 144, but may operate on the basis of a control signal from the robot arm control device 143.

C. Control System of Medical Robot Device

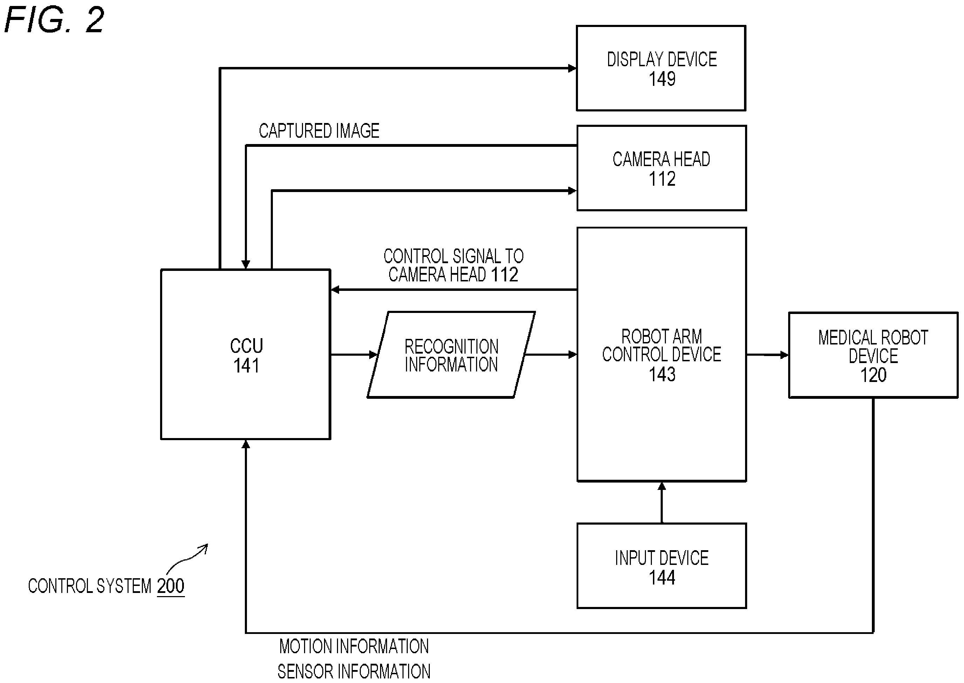

Fig. 2 schematically illustrates a configuration example of a control system 200 that controls the operation of the medical robot device 120 in the endoscopic surgery system 100.

The CCU 141 performs image processing on the image signal transmitted from the camera head 112. The image processing includes, for example, signal processing such as development processing, high image quality processing (band coordination processing, super-resolution processing, noise reduction (NR) processing, camera shake correction processing, and the like), and enlargement processing (electronic zoom processing). Furthermore, an image processing unit 212 performs detection processing on an image signal for performing auto exposure (AE), auto focus (AF), and auto white balance (AWB).

The CCU 141 includes, for example, a processor such as a CPU or a GPU, a local memory thereof, and the like, and executes the above-described image processing and detection processing by the processor executing a predetermined program loaded in the local memory. Furthermore, in a case where the image processing unit 212 includes a plurality of GPUs, information regarding an image signal may be appropriately divided, and image processing may be performed in parallel by the plurality of GPUs.

Furthermore, the CCU 141 receives a captured image of a surgical site by the endoscope 110 from the camera head 112, receives motion information of the robot arm and sensor information of the robot arm from the medical robot device 120, recognizes an image of a medical instrument included in the captured image of the endoscope 110 or an environment in a field of view of the endoscope 110, and outputs instrument recognition information and environment recognition information. The instrument recognition information is a type of the medical instrument (for example, forceps, pneumoperitoneum tube, energy treatment tool, tweezers, retractor, and the like) recognized in the field of view of the endoscope 110, a position and a posture of each instrument, an operation state (for example, in the case of forceps, an open/closed state is obtained, and in the case of an energy treatment tool, an energy output state is obtained), and the like. Furthermore, the environment recognition information is information indicating the environment of the operative field, such as depth information, environment map information, arrangement information of organs and instruments in a space, a material of each object (such as an organ or a metal), and the like. Note that the CCU 141 does not necessarily need to output two types of recognition information of the instrument recognition information and the environment recognition information as the image recognition result, and may output three or more types of recognition information separately, or may output all the recognition results collectively as one piece of recognition information.

The robot arm control device 143 supplies the target control command-related information to the CCU 141 and the medical robot device 120. Note that, in the present specification, for example, a plurality of types of target command values such as a camera target position, a posture, a speed, an acceleration, a gaze point, a line-of-sight vector (target object position, distance, vector posture) of the endoscope, an electronic cut-out position of a captured image, a distance, and a joint angle and a joint angular velocity of each joint of a robot arm supporting the endoscope is collectively referred to as target command-related information. The robot arm control device 143 calculates target command-related information including target command values such as a joint angle and a joint angular velocity of each joint of the robot arm on the basis of the instrument recognition information and the environment recognition information obtained by the CCU 141 performing image recognition on the captured image of the endoscope 110, and outputs a control signal to the medical robot device 120. Furthermore, the robot arm control device 143 calculates target command-related information including target command values such as magnification and focus of the captured image on the basis of the instrument recognition information and the environment recognition information, generates a control signal for controlling the drive of the camera head 112, and outputs the control signal to the CCU 141. In a case where a user (for example, a surgeon, a doctor, an assistant, and the like) inputs an imaging condition via the input device 144, the robot arm control device 143 generates a control signal to the medical robot device 120 or the camera head 112 on the basis of the user input. Furthermore, in a case where the endoscope 110 is equipped with an AE function, an AF function, and an AWB function, the robot arm control device 143 calculates the optimum exposure value, focal length, and white balance on the basis of the result of the detection processing by the CCU 141, and outputs control signals for AE, AF, and AWB for the camera head 112 to the CCU 141.

The medical robot device 120 operates the robot arm on the basis of a control signal from the robot arm control device 143, and outputs motion information of the robot arm and sensor information detected by a sensor mounted on the medical robot device 120 to the robot arm control device 143. Furthermore, the camera head 112 receives a control signal from the robot arm control device 143 via the CCU 141, and outputs a captured image of the surgical site captured by the endoscope 110 to the CCU 141.

The CCU 141 causes the display device 149 to display the captured image of the endoscope 110. Furthermore, the control unit 213 may generate surgery support information based on the image recognition result as described above, and display the surgery support information in a superimposed manner when displaying the image of the surgical site captured by the endoscope 110 on the display device 149. The surgeon 101 can proceed with the surgery more safely and reliably on the basis of the surgery support information presented together with the image of the surgical site.

According to the present disclosure, a determination basis at the time of automatically operating the medical robot device 120 and information regarding uncertainty or reliability of data used for the automatic operation are presented as surgery support information, and this point will be described later in detail.

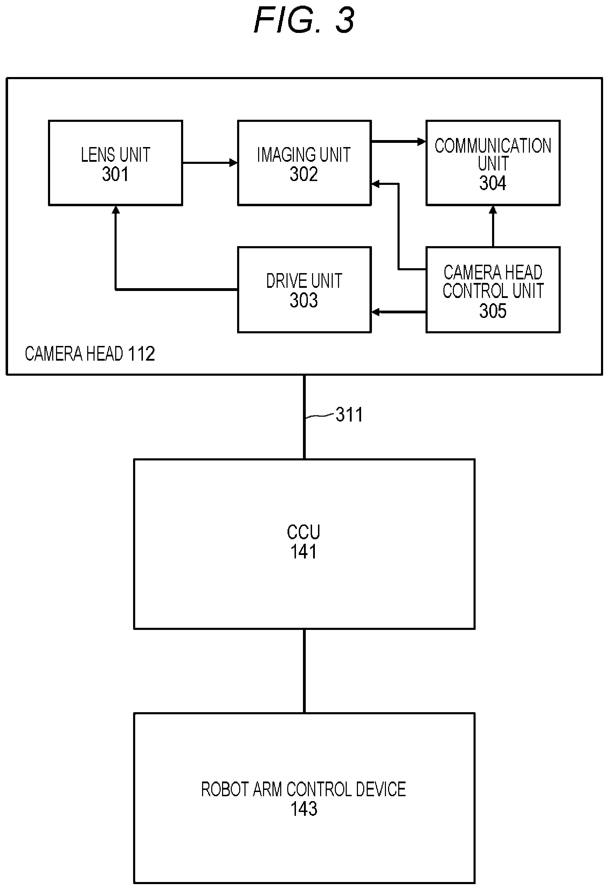

Fig. 3 illustrates an internal configuration example of the camera head 112. The camera head 112 includes a lens unit 301, an imaging unit 302, a drive unit 303, and a camera head control unit 305.

The lens unit 301 is an optical system disposed at a connection portion with the lens barrel 111. Observation light taken in from the distal end of the lens barrel 111 is guided to the camera head 112 and enters the lens unit 301. The lens unit 301 is configured by combining a plurality of optical lenses including a zoom lens and a focus lens. Optical characteristics of the lens unit 301 are adjusted such that incident light forms an image on a light receiving surface of an imaging element of the imaging unit 302. Furthermore, the zoom lens and the focus lens are movable in position on the optical axis in order to adjust the magnification and focus of the captured image.

The imaging unit 302 includes a light receiving element and is arranged at a subsequent stage of the lens unit 301. The light receiving element may be, for example, an imaging element such as a complementary metal oxide semiconductor (CMOS), a time of flight (ToF) sensor, or the like. The imaging unit 302 may be disposed immediately after the objective lens inside the lens barrel 111 instead of inside the camera head 112. The imaging unit 302 photoelectrically converts the observation light formed on the light receiving surface of the light receiving element by the lens unit 301, generates a pixel signal corresponding to the observation image, and outputs the pixel signal to the communication unit 303.

The light receiving element may be, for example, an imaging element having the number of pixels corresponding to a resolution of 4K (the number of horizontal pixels 3840 $\times$ the number of vertical pixels 2160), 8K (the number of horizontal pixels 7680 $\times$ the number of vertical pixels 4320), or a square 4K (the number of horizontal pixels 3840 or more $\times$ the number of vertical pixels 3840 or more). When a high-resolution image of the surgical site is obtained using such an imaging element capable of coping with imaging of a high-resolution image, the surgeon 101 can grasp the state of the surgical site with a high-definition image on the screen of the display device 149, and the surgery can proceed more smoothly. Furthermore, the imaging unit 302 may include a pair of imaging elements so as to support 3D display. By performing the 3D display, the surgeon 101 can more accurately grasp the depth of the living tissue in the surgical site, and can more smoothly progress the surgery.

The drive unit 303 includes an actuator, and moves the zoom lens and the focus lens of the lens unit 301 by a predetermined distance in the optical axis direction under the control of the camera head control unit 305 to adjust the magnification and focus of the image captured by the imaging unit 302.

The communication unit 304 includes a communication device that transmits and receives various types of information to and from the CCU 141 and the robot arm control device 143. The communication unit 304 is used to transmit the image signal obtained from the imaging unit 302 to the CCU 141 via a transmission cable 311. In order for the surgeon 101 to perform surgery more safely and reliably while observing the captured image of the surgical site, it is necessary to display a moving image of the surgical site in real time. Therefore, in order to transmit the image signal obtained from the imaging unit 302 with low latency, the communication unit 304 preferably performs optical communication. In the case of performing optical communication, the communication unit 304 includes an electro-optical conversion module, converts an electric signal into an optical signal, and transmits the optical signal to the CCU 141 via a transmission cable (optical fiber) 311.

Furthermore, the communication unit 304 receives a control signal for controlling driving of the camera head 112 from the robot arm control device 143 side via the transmission cable 312, and supplies the control signal to the camera head control unit 305. The control signal includes information regarding the frame rate of the captured image, information regarding the exposure at the time of imaging, and information regarding the magnification and focus of the captured image. The endoscope 110 may be equipped with AE, AF, and AWB functions. In this case, imaging conditions such as a frame rate, an exposure value, a magnification, and a focus may also be automatically set by the robot arm control device 143 via the CCU 141.

The camera head control unit 305 controls driving of the camera head 112 on the basis of a control signal received from the CCU 141 via the communication unit 304. For example, the camera head control unit 305 controls driving of the imaging element of the imaging unit 302 on the basis of a control signal that specifies a frame rate or an exposure value. Furthermore, the camera head control unit 305 adjusts the positions of the zoom lens and the focus lens of the lens unit 301 in the optical axis direction via the drive unit 303 on the basis of a control signal for designating the magnification and the focus of the captured image. Furthermore, the camera head control unit 305 may have a function of storing information for identifying the lens barrel 111 and the camera head 112.

The transmission cable 311 connecting the camera head 112 and the CCU 141 may be an electric signal cable compatible with electric signal communication, an optical fiber compatible with optical communication, or a composite cable thereof. Alternatively, the camera head 112 and the CCU 141 may be wirelessly connected instead of a wired cable. In a case where the camera head 112 and the CCU 141 are connected by wireless communication, it is not necessary to lay the transmission cable 220 in the operating room, and the movement of the medical staff such as the surgeon 101 and an assistant is not hindered by the transmission cable 311.

Note that by disposing a part of the camera head 112 such as the lens unit 301 and the imaging unit 302 in a sealed structure having high confidentiality and waterproofness, resistance to autoclave sterilization can be imparted.

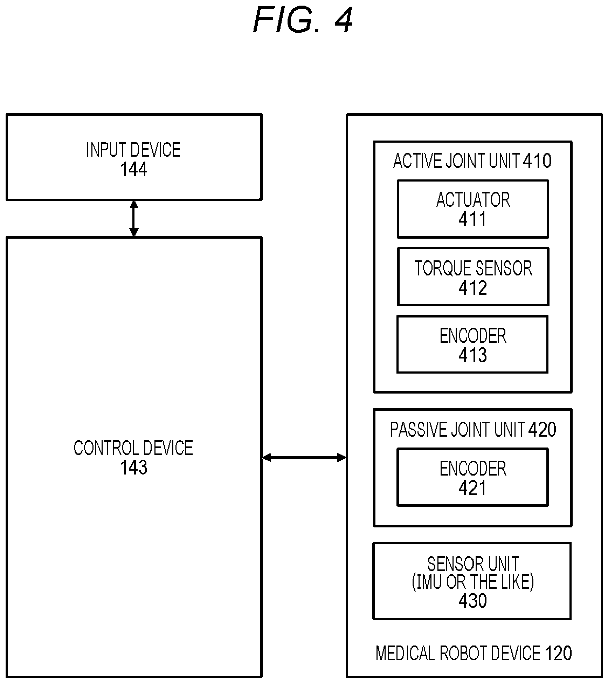

Fig. 4 schematically illustrates a functional configuration example of the medical robot device 120.

The medical robot device 120 is, for example, a robot arm including a multilink structure having six or more degrees of freedom. The robot arm has a structure in which the endoscope 110 is supported at a distal end portion. For example, the distal end portion of the robot arm may have a structure in which orthogonal rotation axes with three degrees of freedom for determining the posture of the endoscope 110 are intensively arranged. In Fig. 4, it is assumed that the function of the medical robot device 120 is abstracted, joints connecting links are classified into two types of an active joint unit 410 and a passive joint unit 420, and the medical robot device 120 includes a sensor unit 440.

The active joint unit 410 includes an actuator 411 such as a rotary motor that drives a joint, a torque sensor 412 that detects torque acting on the joint, and an encoder 413 that measures a rotation angle of a joint. Furthermore, the passive joint unit 420 includes an encoder 421 that measures a joint angle. The sensor unit 430 includes various sensors disposed outside the joint unit, such as an inertial measurement unit (IMU) and a contact sensor that detects a contact force acting on a medical instrument attached to the distal end of the robot arm.

The robot arm control device 143 generates a target operation of the medical robot device 120 on the basis of the recognition information output from the CCU 141 and a user's instruction input via the input device 144, and controls driving of the medical robot device 120 according to a predetermined control method such as position control and force control. Specifically, the robot arm control device 143 calculates a control amount of the actuator 411 of the active joint unit 410 according to a predetermined control method and supplies a drive signal. The robot arm control device 143 includes, for example, a processor such as a CPU, a local memory thereof, and the like, and executes a predetermined program loaded in the local memory by the processor.

Between the robot arm control device 143 and the medical robot device 120 may be an electric signal cable compatible with electric signal communication, an optical fiber compatible with optical communication, or a composite cable thereof. Furthermore, it may be included in the above-described transmission cable 311. Furthermore, the robot arm control device 143 and the medical robot device 120 may be connected wirelessly instead of a wired cable.

D. Control System Using Neural Network Model

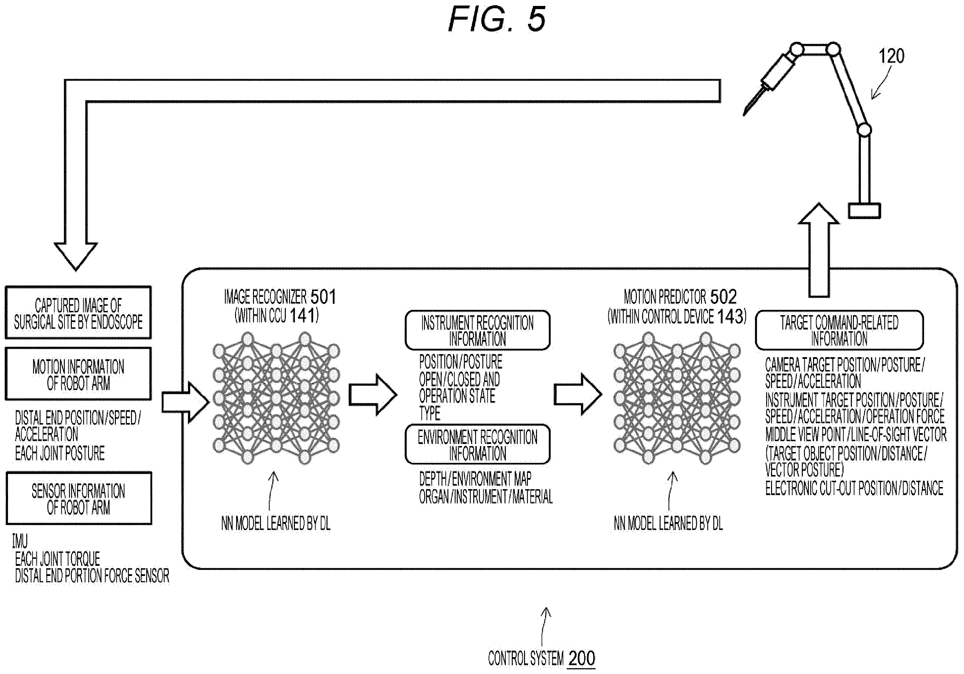

Fig. 5 illustrates a configuration example of a control system 200 using a neural network model.

In Fig. 5, the CCU 141 performs image recognition of the captured image of the surgical site by the endoscope 110 using an image recognizer 501 including a neural network model learned by deep learning. At the time of image recognition, the image recognizer 501 also uses motion information of the robot arm and sensor information of the robot arm from the medical robot device 120 as input data.

Here, the motion information of the robot arm input to the image recognizer 501 includes information on the position, speed, and acceleration of the medical instrument such as the endoscope 110 supported at the distal end by the robot arm, and the posture of each joint of the robot arm (joint angle measured by the encoder installed on the rotation axis of the joint). Furthermore, the sensor information of the robot arm input to the image recognizer includes information such as acceleration measured by the IMU mounted on the medical robot device 120, torque information acting on each joint, and information such as an external force acting on the medical instrument supported at the distal end of the robot arm.

Then, the image recognizer 501 performs image recognition of the medical instrument included in the captured image of the endoscope 110 and the environment in the field of view of the endoscope 110, and outputs the instrument recognition information and the environment recognition information. The image recognizer 501 recognizes the type of the medical instrument (for example, forceps, pneumoperitoneum tube, energy treatment tool, tweezers, retractor, and the like), the position and posture of each instrument, and the operation state (for example, in the case of forceps, an open/closed state is obtained, and in the case of an energy treatment tool, an energy output state is obtained) recognized in the field of view of the endoscope 110 as the instrument recognition information. Furthermore, the image recognizer 501 recognizes, as the environment recognition information, depth information of an organ or a medical instrument included in the captured image in the field of view of the endoscope 110 (including a shape of the organ or the instrument), an environment map in a surgical site (for example, environment map creation using simultaneous localization and mapping (SLAM) technology), a type of the organ, a type of the medical instrument, a material of each object included in the captured image, and the like. Furthermore, the image recognizer 501 recognizes, for example, each object such as an organ or a medical instrument included in the image of the surgical site and a material thereof, depth information of each object, and an environment map as the environment recognition information. Note that the image recognizer 501 does not necessarily need to output two types of recognition information of the instrument recognition information and the environment recognition information as the image recognition result, and may output three or more types of recognition information separately, or may output all the recognition results collectively as one piece of recognition information.

Furthermore, in Fig. 5, the robot arm control device 143 predicts and outputs the target command-related information to the medical robot device 120 on the basis of the recognition information of the instrument recognition information and the environment recognition information output from the CCU 141 using a motion predictor 502 including a neural network model learned by deep learning. The motion predictor 502 predicts various target command values such as a camera target position, a posture, a speed, an acceleration, a gaze point, a line-of-sight vector (target object position, distance, vector posture), an electronic cut-out position of a captured image, and a distance of the endoscope 110, for example, as the target command-related information. In a case where the medical robot device 120 supports a medical instrument other than the endoscope 110 at the distal end, the motion predictor 502 predicts the target position, posture, speed, acceleration, and operation force of the instrument as the target command-related information. The robot arm control device 143 calculates a target joint angle, a joint angular velocity, and a joint angular acceleration of each joint of the robot arm by inverse kinematics calculation on the basis of the information of the target position, posture, speed, and acceleration of the medical instrument supported by the distal end of the robot arm such as the endoscope 110 predicted by the motion predictor 502, and outputs a command value to the medical robot device 120.

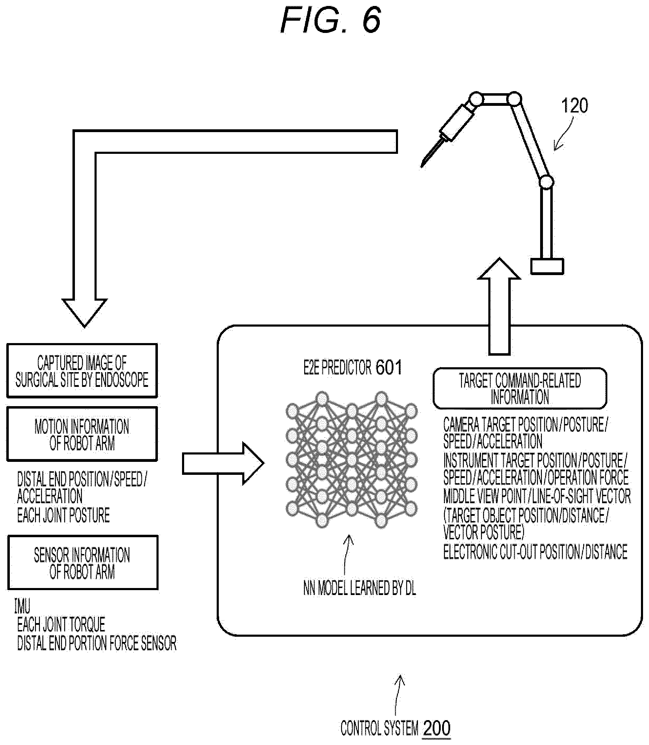

Furthermore, the image recognizer 501 and the motion predictor 502 may not be configured by individual neural network models as illustrated in Fig. 5, but may be configured as a predictor 601 including a neural network model of E2E by integrating image recognition and motion prediction as illustrated in Fig. 6.

As illustrated in Figs. 5 and 6, for example, by performing motion prediction of the robot arm using a neural network model learned by deep learning, real-time control of the medical robot device 120 can be implemented in consideration of various input parameters. Therefore, by introducing the medical robot device 120 that supports an endoscope in an endoscopic surgery and controlling the operation and processing of the robot on the basis of inference by deep learning, it is possible to reduce the costs such as labor costs of the operator and to improve the accuracy and safety of the control technology of the endoscope.

E. Control System for Presenting Determination Basis

As described in section D described above, when deep learning is used for operation control of the medical robot device 120, it is necessary to clarify the validity of the determination basis. In general, deep learning is compared to a black box because it is difficult to understand the basis of determination. Therefore, in the present disclosure, the basis of the determination in the operation prediction of the medical robot device 120 using deep learning is made clear. Therefore, the doctor can smoothly perform the surgery while confirming that the operation of the medical robot device 120 is not different from his/her own determination and the determination basis of the operation prediction.

E-1. Control System (1) for Presenting Determination Basis

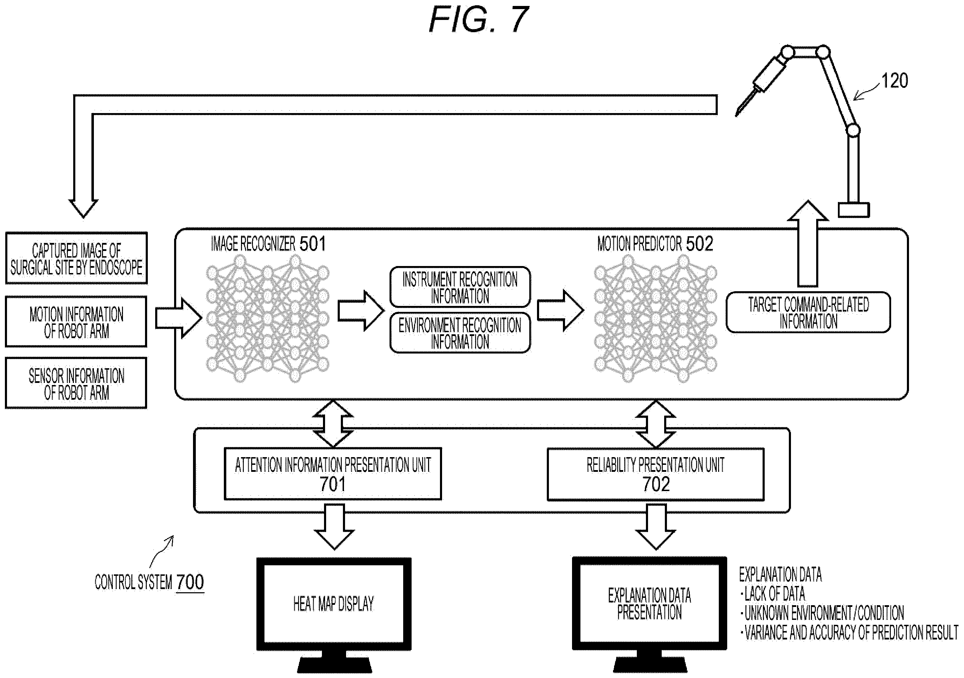

Fig. 7 illustrates a configuration example of a control system 700 in which the image recognizer 501 and the motion predictor 502 are configured by individual neural network models, and a determination basis of motion prediction by the neural network model is presented. Note that the image recognizer 501 and the motion predictor 502 may be collectively referred to as a "learner". The control system 700 incorporates an attention information presentation unit 701 and a reliability presentation unit 702 in the system configuration illustrated in Fig. 5. The attention information presentation unit 701 presents, for example, information regarding a target object that the neural network model has paid attention to when estimating the target command-related information. The attention information presentation unit 701 presents information of medical instruments of which attention is paid, such as a gripping tool (grasper) and scissors used for surgery, for example. Furthermore, the reliability presentation unit 702 presents the reliability of how correctly the medical robot device 120 is likely to move on the basis of the target command-related information output by the neural network model with a statistical score representing the certainty (accuracy) such as the probability indicating the accuracy of the determination result and the variance indicating the dispersion in the solution, and the like. Hereinafter, the attention information presentation unit 701 and the reliability presentation unit 702 will be described.

The attention information presentation unit 701 presents information of which attention is paid when the motion predictor 502 determines the target command-related information in the image input to the control system 700. Specifically, the image input to the control system 700 is an operative field image captured by the endoscope. The attention information presentation unit 701 uses an algorithm such as gradient-weighted class activation mapping (Grad-Cam) that visualizes a determination basis in the image classification problem or local interpretable model-agnostic explanations (LIME) that interprets a machine learning model to present information of which attention is paid when the image recognizer 501 estimates the instrument recognition information or the environment recognition information.

Grad-Cam is known as a technology for visualizing information that is a basis of determination of a deep-learned neural network model and displaying the information on a heat map. The attention information presentation unit 701 to which Grad-Cam is applied displays a heat map indicating which part of the image, which target object in the image, or the like is focused on to output or estimate the target command-related information from the input image, the motion information of the robot, and the sensor information of the robot.

The operation principle of Grad-Cam is to visualize information serving as a determination basis by displaying a place having a large input gradient with respect to the final layer of a target convolutional neural network (CNN) (in this case, the motion predictor 502). The flow of processing of Grad-Cam includes reading of input data, reading of a model, a prediction class of an input image, loss calculation of the prediction class, calculation of back propagation to a final convolution layer, and weight calculation of each channel in the final convolution layer (calculation of gradient for each channel by global average pooling that is average processing). Here, assuming that the gradient $y^c$ of the class c is the feature map activation $A_k$, the weight $\alpha^c_k$ of the importance level of the neuron is given as in the following formula (1).
[Mathematical Formula 1]

$$\alpha^c_k = \frac{1}{Z} \sum_i \sum_j \frac{\partial y^c}{\partial A^k_{ij}} \qquad \cdots (1)$$

The weight for each channel is added to the forward propagation output of the final convolution layer, and Grad-Cam is calculated as in the following formula (2) through the activation function ReLU.
[Mathematical Formula 2]

$$L^c_{Grad-Cam} = ReLU\left(\sum_k \alpha^c_k A^k\right) \qquad \cdots (2)$$

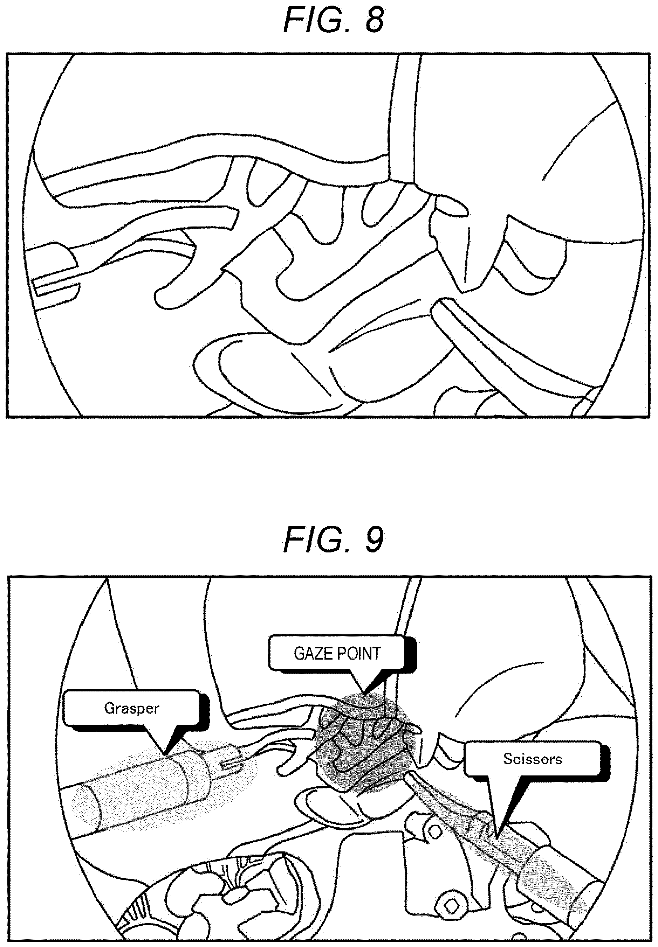

Fig. 8 illustrates a monitor image of the operative field. The monitor image of the operative field is an image electronically cut out from the captured image of the endoscope 110. The entire monitor image or the captured image of the endoscope 110 is an input image to the control system 700. Furthermore, Fig. 9 illustrates an image in which a part focused when the control system 700 outputs the target command-related information is displayed on a heat map, the part being presented by the attention information presentation unit 701 to which the Grad-Cam is applied. The monitor image illustrated in Fig. 8 is a captured image of the endoscope 110 or an operative field image displayed on the display device 149. Meanwhile, Fig. 9 is an image with a wide angle of view having a wider field of view than the operative field image of Fig. 8. The wide angle image may be an image captured by the endoscope 110 while zooming out in the same line-of-sight direction as the operative field image in Fig. 8, or may be an environment image recognized by the image recognizer 501 using SLAM technology or the like. The doctor can observe the surgical site in detail from the operative field image illustrated in Fig. 8, and can also grasp the basis of the target command for the medical robot device 120 by the control system 700 in a bird's-eye view from the wide angle image illustrated in Fig. 9.

In the wide angle image illustrated in Fig. 9, a gaze point observed when the motion predictor 502 outputs a target command, and medical instruments such as a gripping tool "grasper" and "scissors" recognized by the image recognizer 501 are displayed in a heat map. In the image illustrated in Fig. 9, in addition to the heat map display, the attention information is presented by displaying the meta information of each object of which attention is paid with characters or emphasizing using the size or color of the characters.

The presentation image of the attention information as illustrated in Fig. 9 is displayed on the display device 149 together with the captured image of the endoscope 110 illustrated in Fig. 8. The display form is arbitrary. For example, a region for displaying a presentation image of an attention image may be provided in a format such as a picture-in-picture (PinP) in a screen for displaying a captured image of the endoscope 110 as a main video, and the captured image of the endoscope 110 and the presentation image of the attention information may be simultaneously displayed. The captured image of the endoscope 110 and the presentation image of the attention information may be alternately displayed on the screen. Alternatively, in addition to the main display that displays the captured image of the endoscope 110, a sub display that displays the presentation image of the attention information may be added.

Therefore, on the basis of the wide angle image illustrated in Fig. 9, the surgeon or the doctor can smoothly perform the surgery while visually checking which information the control system 700 focuses on to output the target command-related information to the medical robot device 120 (that is, in cooperation with the control system 700) and confirming that the operation of the medical robot device 120 is not different from his/her own determination.

Note that the attention information presentation unit 701 may present information of which attention is paid when the control system 700 determines the target command-related information by using a technology other than Grad-Cam, such as LIME, for example. Furthermore, the attention information presentation unit 701 may present the attention information in a display format other than the heat map, or may present the long length information in combination with another output format such as heat map display and voice guidance.

Next, the reliability presentation unit 702 will be described. The reliability presentation unit 702 presents information for explaining the reliability such as how correctly the medical robot device 120 is likely to move on the basis of the target command-related information output by the motion predictor 702. Specifically, the reliability presentation unit 702 presents a numerical value indicating, for example, lack of data and a degree of influence of data when the motion predictor 502 outputs the target command-related information to the medical robot device 120, an unknown environment/condition, a probability indicating accuracy of a prediction result, a statistical score indicating certainty (accuracy) such as variance indicating dispersion in solutions, as data for explaining uncertainty or reliability of the target command-related information. The reliability presentation unit 702 estimates the uncertainty or the reliability of the target command-related information using, for example, a Bayesian deep neural network (DNN), and details of this point will be described later.

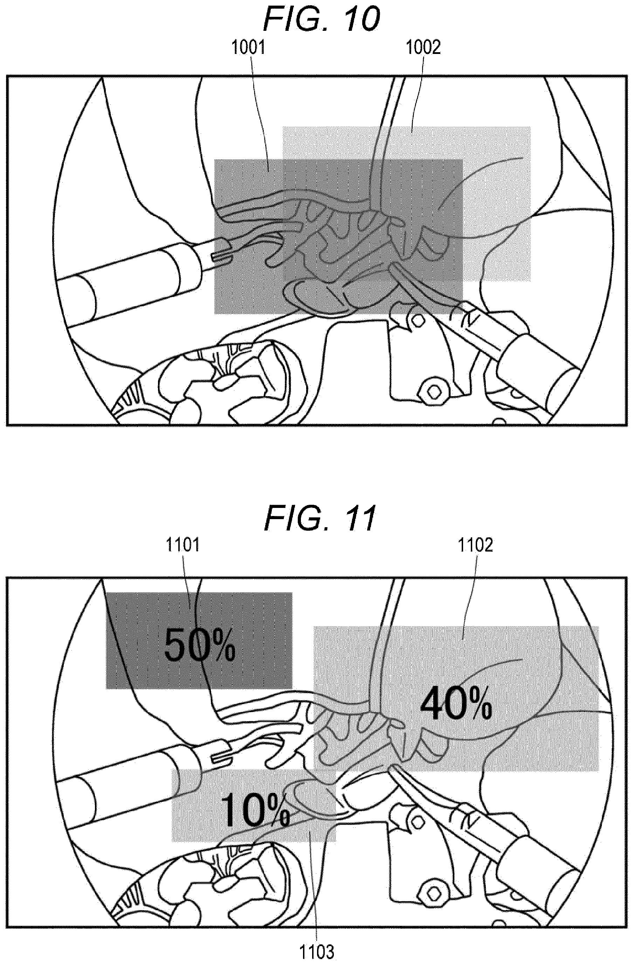

Fig. 10 illustrates an image for presenting the target command-related information to the medical robot device 120 presented by the reliability presentation unit 702. Fig. 10 is an image with a wide angle of view having a wider field of view than the operative field image of Fig. 8. The wide angle image may be an image captured by the endoscope 110 while zooming out in the same line-of-sight direction as the operative field image in Fig. 8, or may be an environment image recognized by the image recognizer 501 using SLAM technology or the like. In the example illustrated in Fig. 10, the current display position (electronic cut-out position of the image captured by the endoscope 110 supported at the current position of the arm of the robot device 120 or the captured image) of the endoscopic image indicated by reference numeral 1001 and the next display position indicated by reference numeral 1002 are simultaneously displayed in the wide angle image. Here, the next display position 1002 is a captured image of the endoscope 110 at a position where the arm of the medical robot device 120 is operated on the basis of the target command-related information. The doctor can observe the surgical site in detail from the operative field image illustrated in Fig. 8, and can smoothly perform the surgery while confirming that the surgical site image displayed when the medical robot device 120 is operated according to the target command-related information output from the control system 700 is not different from his/her own determination on the basis of the presentation image of the target command-related information illustrated in Fig. 10.

Furthermore, Fig. 11 illustrates another example of an image that presents an explanation of the uncertainty or the reliability of the target command-related information to the medical robot device 120, presented by the reliability presentation unit 702. Fig. 11 is an image with a wide angle of view having a wider field of view than the operative field image of Fig. 8 (same as above). In the example illustrated in Fig. 11, it is assumed that three patterns of candidates of the target command-related information are output from the motion predictor 502, and captured images 1101, 1102, and 1103 of the endoscope 110 at positions where the arm of the medical robot device 120 is operated on the basis of the respective candidates are displayed together with the accuracy (50%, 40%, 10%) of the respective candidates. Although not illustrated in Fig. 11, a heat map as illustrated in Fig. 9 may be displayed in a superimposed manner. The doctor can observe the surgical site in detail from the operative field image illustrated in Fig. 8, and can smoothly perform the surgery while confirming with which candidate the surgical site image displayed when the medical robot device 120 is operated according to the target command-related information output from the control system 700 is not different from his/her own determination on the basis of the explanatory presentation image of the uncertainty or the reliability illustrated in Fig. 11. For example, the doctor may check an image presenting an explanation of uncertainty or reliability as illustrated in Fig. 11 so that any candidate can be selected or corrected by a voice command. Furthermore, an image presenting an explanation of uncertainty or reliability as illustrated in Fig. 11 may be displayed on a touch panel so that a doctor can select or correct any candidate by touching the screen.

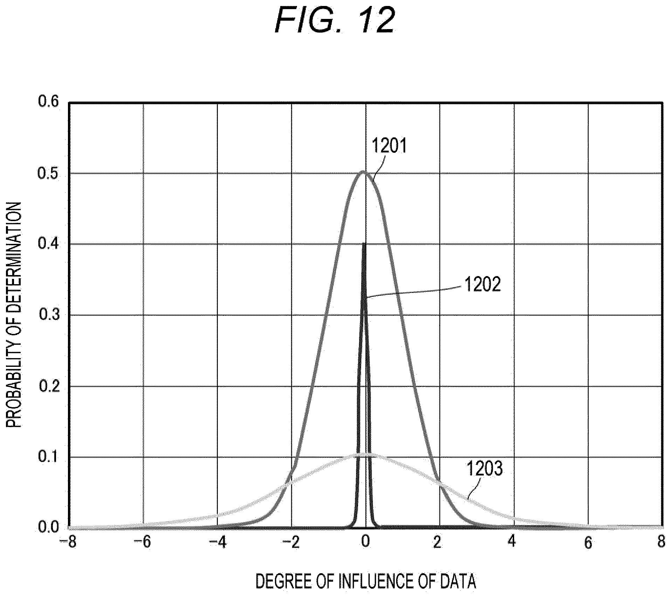

Furthermore, Fig. 12 illustrates still another example of an image that presents an explanation of uncertainty or reliability of the target command-related information to the medical robot device 120, presented by the reliability presentation unit 702. Also in the example illustrated in Fig. 12, it is assumed that three patterns of candidates for the target command-related information are output from the motion predictor 502, and the determination probability of each candidate indicated by reference numeral 1201 to 1203 and the degree of influence of data are plotted in a graph. However, in Fig. 12, normalization is performed such that the sum of the probabilities of determination of the respective candidates 1201 to 1203 becomes 1. The first candidate 1201 has the maximum determination probability of 0.5, but has a large degree of influence of data, in other words, a large dispersion due to lack of data. On the other hand, the second candidate 1202 has a determination probability of 0.4 lower than that of the first candidate 1201, but has a small degree of influence of data, in other words, a small dispersion in data due to lack of data. Furthermore, in the third candidate 1203, the determination probability is 0.1, which is the lowest, and the degree of influence of data is also large. When the explanation of the uncertainty or the reliability is presented on the basis of the image as illustrated in Fig. 12, the doctor can not only simply compare the determination probabilities of each candidate 1201 to 1203, but also determine on the basis of which candidate target command the medical robot device 120 should be operated in consideration of the uncertainty due to the lack of data. Furthermore, with respect to a candidate having a large data uncertainty such as the candidate 1201, the control system 700 may autonomously search for data that compensates for the uncertainty due to lack of data to reduce the uncertainty or improve the reliability.

Note that the attention information presentation unit 701 may estimate information of which attention is paid when the motion predictor 502 determines the target command-related information by using a learned neural network model. Furthermore, the reliability presentation unit 702 may estimate the uncertainty or the reliability of the target command-related information output by the motion predictor 702 by using a learned neural network model. The neural network used by the attention information presentation unit 701 and the reliability presentation unit 702 may be a neural network independent of the image recognizer 501 and the motion predictor 502, or may be a neural network incorporating at least some neurons of the image recognizer 501 and the motion predictor 502.

Furthermore, Fig. 7 illustrates a configuration example of the control system 700 in which the attention information presentation unit 701 and the reliability presentation unit 702 are configured as individual functional modules. However, the attention information presentation unit 701 and the reliability presentation unit 702 can be configured as one functional module together. For example, the attention information presentation unit 701 and the reliability presentation unit 702 can be configured as a neural network model of E2E.

E-2. Bayesian DNN

Next, the Bayesian DNN used by the reliability presentation unit 703 to estimate the uncertainty or the reliability of the target command-related information will be described. The uncertainty of the determination result in the deep-learned neural network model can be divided into two types: aleatoric uncertainty and epistemic uncertainty.

The former case of aleatoric uncertainty is caused by noise due to the observing environment or the like, and is not caused by lack of data. For example, a hidden and invisible image (occlusion) or the like corresponds to the aleatoric uncertainty. Since a mouth of a face of a masked person is originally hidden by the mask, it cannot be observed as data. In an operative field image, a part of an organ hidden by a surgical tool cannot be observed as data. Meanwhile, the latter case of epistemic uncertainty represents uncertainty due to lack of data. Given the presence of sufficient data, the epistemic uncertainty can be improved.

Although it has been difficult to reveal epistemic uncertainty in the image field, the Bayesian deep learning proposal has made it possible to reveal uncertainty (see, for example, Non Patent Document 1). Bayesian deep learning is configured by combining Bayesian inference and deep learning. By using Bayesian inference, how the estimation result varies can be understood, and thus, uncertainty can be evaluated.

Bayesian deep learning is a method of estimating from a result of variance obtained in inference using a dropout in learning of deep learning. Dropout is a technique used to reduce overfitting by randomly reducing the number of neurons in each layer. The loss function in Bayesian deep learning is given by the following formula (3) according to the role of the dropout.

[Mathematical Formula 3]

$$\mathcal{L}(\theta, p) = -\frac{1}{N} \sum_{i=1}^{N} \log P\left(y_i \middle| f^{\widehat{W_i}}(x_i)\right) + \frac{1-p}{2N} \|\theta\|^2 \quad \cdots (3)$$

N: Data point
p: Dropout probability
$\widehat{W_1}$ : Sample
θ: Parameter of distribution

For a detailed mathematical theory of the above formula, refer to, for example, Non Patent Document 2. In conclusion, using dropout in deep learning is performing Bayesian learning. The value obtained by learning is not deterministic and can be calculated by combining the posterior distribution of weights with the dropout. The variance of the posterior distribution can be estimated from the dispersion in which the plurality of outputs is generated by the plurality of dropout coefficients. The Bayesian deep learning performs sampling from the weight distribution by using the dropout not only at the time of learning but also at the time of inference (Monte Carlo dropout). The uncertainty of the inference result can be obtained by repeating the inference many times for the same input. The network learned using the dropout has a structure in which some neurons are missing. Therefore, when an input image is input and inferred, it is possible to obtain an output that passes through neurons missing by the dropout and is characterized by the weight. Moreover, when a same image is input, it takes different paths in the network to output, so the weighted output is different for each case. That is, the network by the dropout can obtain different output distributions at the time of inference for the same input image. A large variance of the output means that the model has a large uncertainty. The average of the distribution by multiple inferences means a final prediction value, and the variance means uncertainty of the prediction value. Bayesian deep learning represents uncertainty from the variance of the output at the time of this inference.

The input data to the learning in the control system 700 illustrated in Fig. 7 is an input image, motion information of the robot, and sensor information of the robot. The Bayesian deep learning can indicate the uncertainty or reliability of the inference result by the calculation described above. As a result, the fact that the variance of the output is large means that the uncertainty of the model is large (or the reliability is low) due to the lack of data and the prediction limit.

E-4. Operation Procedure

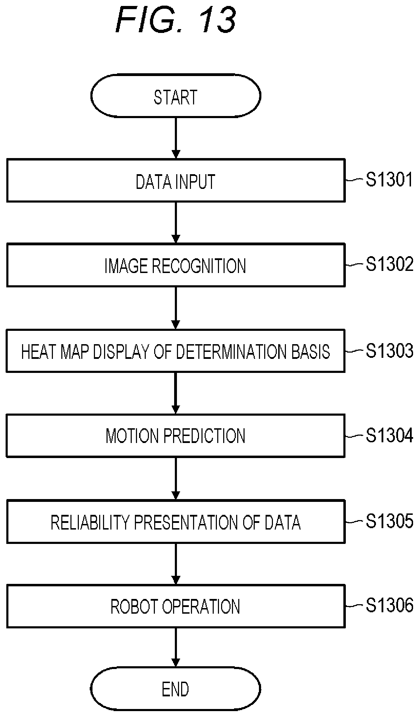

Fig. 13 illustrates a processing procedure for presenting a determination basis of motion prediction by a neural network model to a doctor or the like in the control system 700 illustrated in Fig. 7 in the form of a flowchart.

First, a captured image of the endoscope 110, motion information of the robot, and sensor information of the robot are input to the control system 700 (step S1301) .

The image recognizer 501 performs image recognition on the basis of the data input in step S1301 using the learned neural network model, and outputs the instrument recognition information and the environment recognition information (step S1302).

Then, the attention information presentation unit 701 visualizes the basis of determination when the neural network model used in the image recognizer 501 estimates the instrument recognition information and the environment recognition information by the Grad-Cam algorithm, and performs heat map display (step S1303).

Next, the motion predictor 502 predicts and outputs information related to the target command for the medical robot device 120 on the basis of the recognition information of the instrument recognition information and the environment recognition information output from the image recognizer 501 using the learned neural network model (step S1304).

The reliability presentation unit 702 presents information for explaining how correctly the medical robot device 120 is likely to move on the basis of the target command-related information output by the neural network model used in the motion predictor 502 or the like by the Bayesian DNN (step S1305). In step S1305, the reliability presentation unit 702 presents a numerical value indicating the lack of data, the unknown environment/condition, the variance and accuracy of the prediction result, and the like when the motion predictor 502 outputs the target command-related information to the medical robot device 120, as data for explaining the uncertainty or the reliability of the target command-related information.

Then, the operation of the arm of the medical robot device 120 is controlled on the basis of the target command-related information output by the motion predictor 502 in step S1303 (step S1306). In step S1306, the arm of the medical robot device 120 is driven by a control signal based on the target command-related information output by the motion predictor 502. However, in a case where the operator such as a doctor instructs correction of the operation of the arm on the basis of the information for explaining the uncertainty or the reliability presented in step S1304, the arm of the medical robot device 120 is operated on the basis of the instruction.

E-5. Control System (2) for Presenting Determination Basis

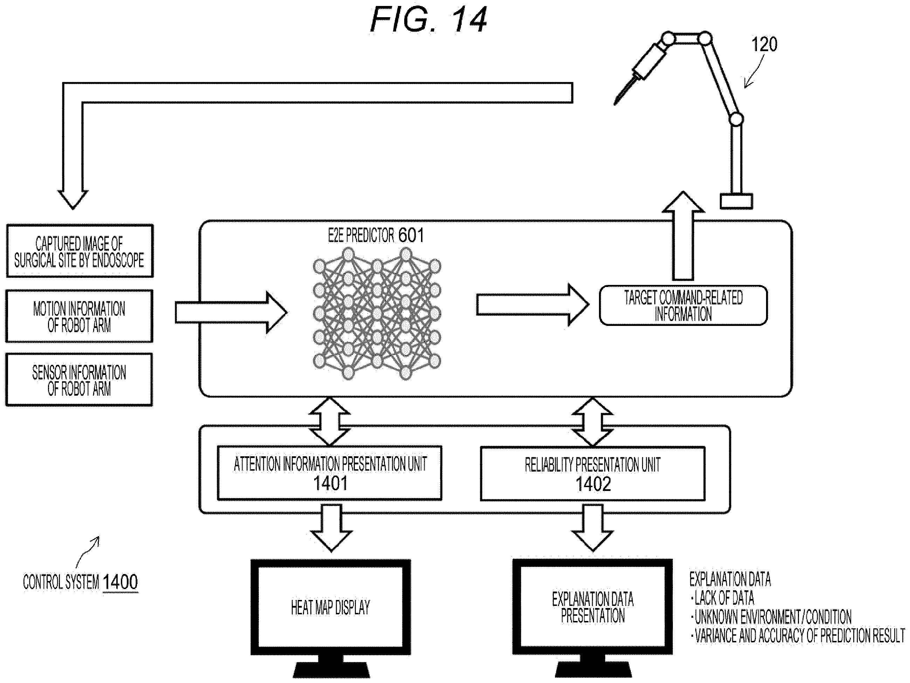

Fig. 14 illustrates a configuration example of a control system 1400 that uses an E2E predictor including a neural network model of E2E by integrating image recognition and motion prediction and presents a determination basis of motion prediction by the neural network model. Note that the E2E predictor may be referred to as a learner. The control system 1400 incorporates an attention information presentation unit 1401 and a reliability presentation unit 1402 in the system configuration illustrated in Fig. 6.

The attention information presentation unit 1401 presents information that the E2E predictor 601 pays attention to when determining the target command-related information. Specifically, the image input to the control system 700 is an operative field image captured by the endoscope. Using an algorithm such as Grad-Cam that visualizes the determination basis in the image classification problem, the attention information presentation unit 701 presents information of which attention is paid when the image recognizer 501 estimates the instrument recognition information and the environment recognition information. The Grad-Cam is as described above.

The attention information presentation unit 1401 presents the attention information in the form illustrated in Fig. 9 with respect to the input image illustrated in Fig. 8, for example. Therefore, on the basis of the wide angle image illustrated in Fig. 9, the doctor can smoothly perform the surgery while visually checking which information the control system 700 focuses on to output the target command-related information to the medical robot device 120 (that is, in cooperation with the control system 700) and confirming that the operation of the medical robot device 120 is not different from his/her own determination.

Furthermore, information for explaining how correctly the medical robot device 120 is likely to move on the basis of the target command-related information output by the E2E predictor 601 is presented. Specifically, the reliability presentation unit 1402 estimates a numerical value indicating lack of data, an unknown environment/condition, variance and accuracy of a prediction result, and the like when the E2E predictor 601 outputs the target command-related information to the medical robot device 120, using, for example, a Bayesian DNN. The Bayesian DNN is as described above.

The reliability presentation unit 1402 presents the explanation of the uncertainty or the reliability of the target command-related information to the medical robot device 120 in a form as illustrated in any of Figs. 10 to 12. The doctor can observe the surgical site in detail from the operative field image illustrated in Fig. 8, and can smoothly perform the surgery while confirming that the surgical site image displayed when the medical robot device 120 is operated according to the target command-related information output from the control system 700 is not different from his/her own determination on the basis of the explanatory presentation image of the uncertainty or the reliability illustrated in Figs. 11 and 12.

E-6. Operation Procedure

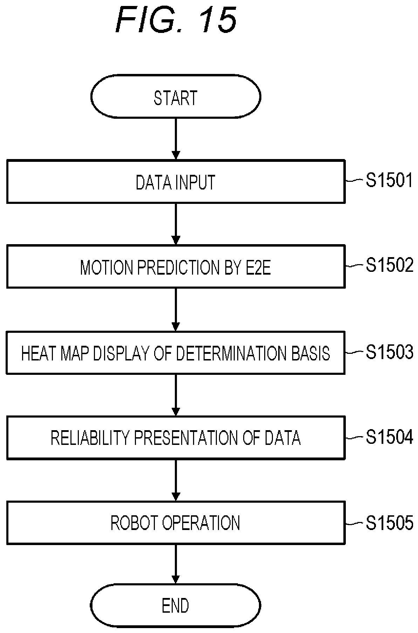

Fig. 15 illustrates a processing procedure for presenting a determination basis of motion prediction by a neural network model to a doctor or the like in the control system 1400 illustrated in Fig. 14 in the form of a flowchart.

First, a captured image of the endoscope 110, motion information of the robot, and sensor information of the robot are input to the control system 1400 (step S1501) .

The E2E predictor 601 predicts and outputs information related to the target command for the medical robot device 120 on the basis of the data input in step S1501 using the learned neural network model (step S1502) .

The attention information presentation unit 1401 visualizes the basis of the determination when the neural network model used in the E2E predictor 601 estimates the target command-related information by the Grad-Cam algorithm, and performs heat map display (step S1503) .

Furthermore, the reliability presentation unit 1402 presents information for explaining how correctly the medical robot device 120 is likely to move on the basis of the target command-related information output by the neural network model used in the E2E predictor 601 by the Bayesian DNN (step S1504). In step S1504, the reliability presentation unit 702 presents a numerical value indicating the lack of data, the unknown environment/condition, the variance and accuracy of the prediction result, and the like when the motion predictor 502 outputs the target command-related information to the medical robot device 120, as data for explaining the uncertainty or the reliability of the target command-related information.

Then, the operation of the arm of the medical robot device 120 is controlled on the basis of the target command-related information output by the motion predictor 502 in step S1502 (step S1505). In step S1505, the arm of the medical robot device 120 is driven by a control signal based on the target command-related information output by the motion predictor 502. However, in a case where the operator such as a doctor instructs correction of the operation of the arm on the basis of the information for explaining the uncertainty or the reliability presented in step S1503, the arm of the medical robot device 120 is operated on the basis of the instruction.

E-4. Presentation Example of Determination Basis

The doctor can smoothly perform the surgery while confirming that the operation of the medical robot device 120 is not different from his/her own determination by viewing the image presenting the determination basis of the target command-related information of the medical robot device 120 output by the control system 700 (or 1400) together with the operative field image captured by the endoscope 110.

The form in which the determination basis is presented is arbitrary. However, it is preferable that the doctor can simultaneously confirm the operative field image and the determination basis during the surgery. For example, a region for displaying a presentation image of a determination basis may be provided in a format such as PinP in a screen for displaying an operative field image by the endoscope 110 as a main video on the display device 149, and the operative field image by the endoscope 110 and the presentation image of the determination basis may be simultaneously displayed. Furthermore, the operative field image by the endoscope 110 and the presentation image of the determination basis may be alternately displayed by using one screen. Alternatively, in addition to the main display that displays an image by the endoscope 110, a sub display that displays the presentation image of the attention information may be added.

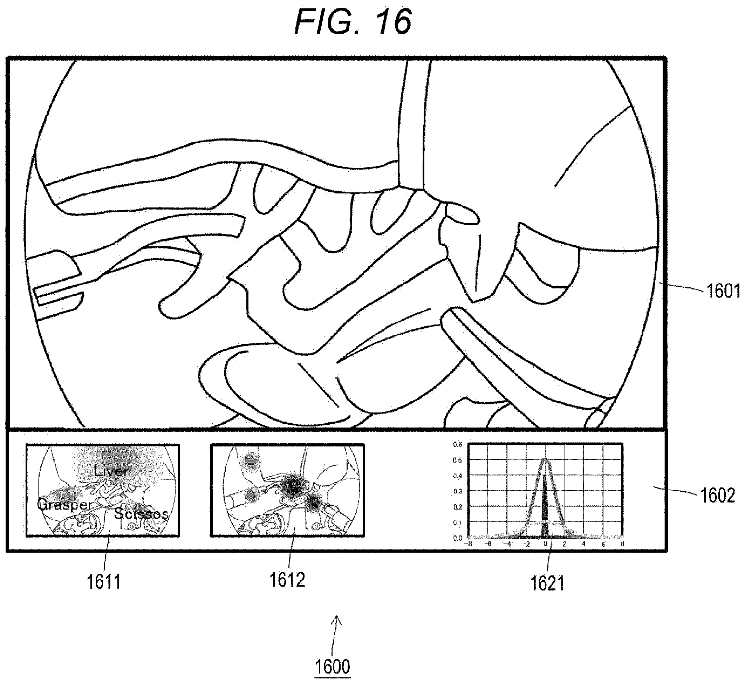

Fig. 16 illustrates an example of a display form in which an operative field image by the endoscope 110 and a presentation image of a determination basis are simultaneously displayed using one screen. In the illustrated example, a main surgery video display unit 1601 that displays an operative field image by the endoscope 110 and an information presentation unit 1602 that presents information regarding a determination basis of the target command-related information to the medical robot device 120 are provided in the screen 1600.

On the main surgery video display unit 1601, an operative field image electronically cut out from an image captured by the endoscope 110 at the current position is displayed. Meanwhile, the information presentation unit 1602 displays a heat map image generated by the attention information presentation unit 701 (or 1401) and an image that presents uncertainty (lack of data, unknown environment/conditions, variance and accuracy of prediction results, and the like) or reliability in motion prediction generated by the reliability presentation unit 702 (or 1402) or the reliability presentation unit 702.

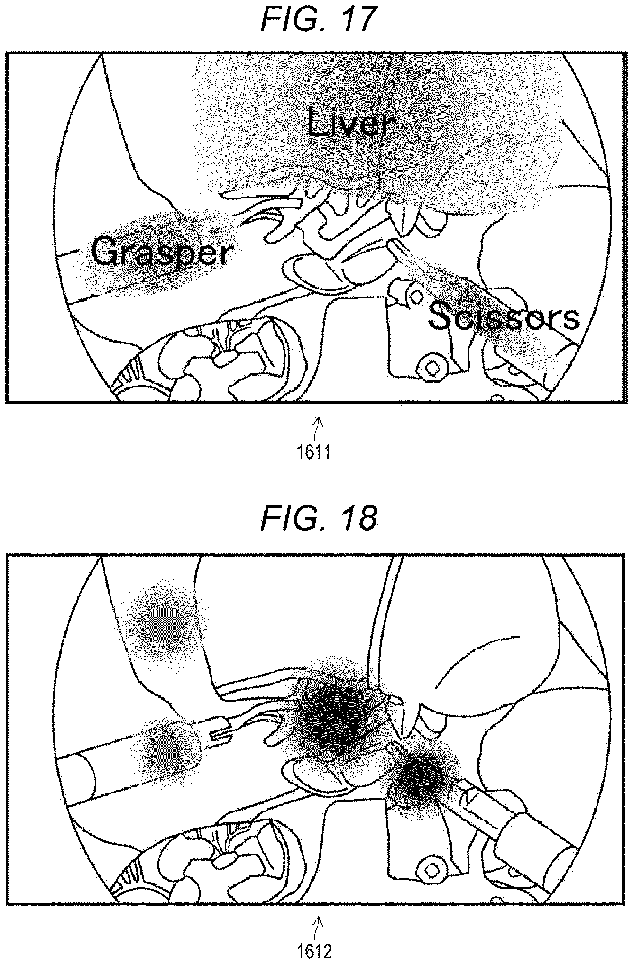

The attention information presentation unit 701 may generate a plurality of types of heat map images indicating the attention information. Furthermore, the reliability presentation unit 702 may generate a plurality of types of presentation images indicating lack of data, unknown environments/conditions, variance and accuracy of prediction results in motion prediction, and the like. Then, a plurality of types of heat map images and a presentation image of uncertainty or reliability may be simultaneously presented to the information presentation unit 1602. In the example illustrated in Fig. 16, a heat map image 1611 in which instruments (grasper and scissors) or an environment (organ (liver) or the like) recognized by the image recognizer 501 is displayed as a heat map, a heat map image 1612 in which a plurality of gaze points observed at the time of motion prediction is displayed as a heat map, and an image 1621 presenting explanation of uncertainty or reliability of the target command-related information are simultaneously displayed on the information presentation unit 1602. For reference, Fig. 17 and Fig. 18 illustrate the heat map image 1611 and the enlarged heat map image 1612 in an enlarged manner, respectively. Furthermore, regarding the explanatory presentation image 1621 of the uncertainty or the reliability, refer to Fig. 12 and the explanation described above.

In this way, by presenting the determination basis by the neural network model in a plurality of forms using the information presentation unit 1602, the doctor can smoothly perform the surgery while accurately confirming in a short time whether or not the operation of the medical robot device 120 is different from his/her determination.

F. Reflection of Determination of Doctor

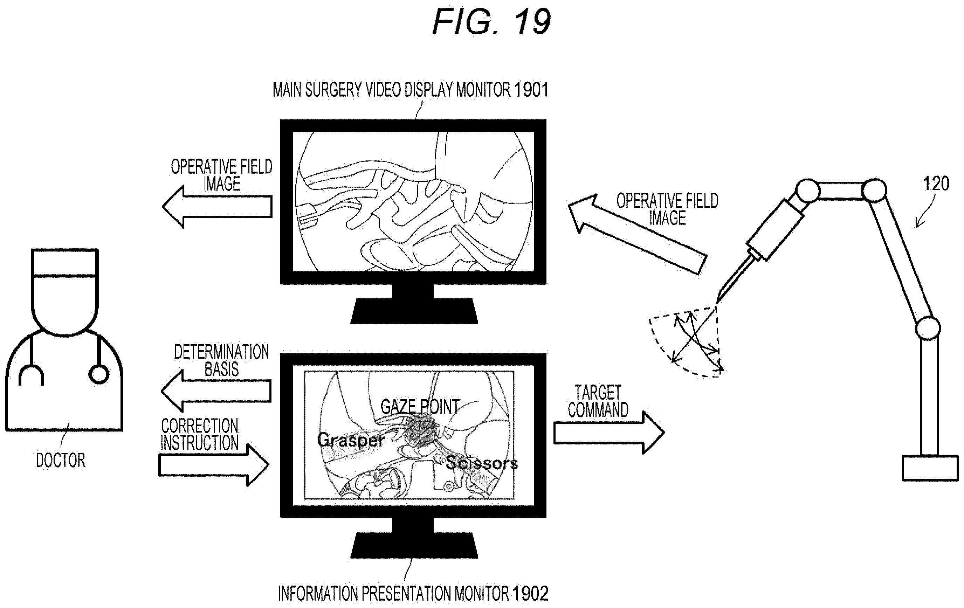

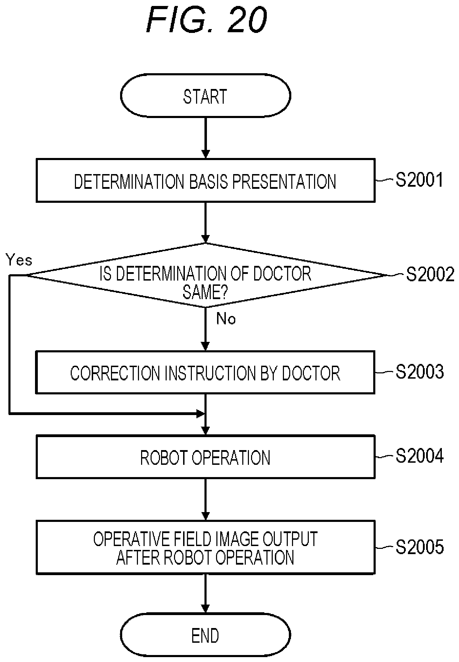

Fig. 19 schematically illustrates a procedure in a case where a doctor performs an endoscopic surgery using the endoscopic surgery system 100 to which the present disclosure is applied. Furthermore, Fig. 20 illustrates an operation procedure in the endoscopic surgery system 100 at the time of surgery in the form of a flowchart.

In Fig. 19, for convenience, the operative field image by the endoscope 110 is displayed on a main surgery video display monitor 1901, and the information regarding the determination basis of the target command-related information to the medical robot device 120 is presented on an information presentation monitor 1902. Note that the number of cases and the number of pieces of learning data handled by a learner (image recognizer 501 and motion predictor 502, or E2E predictor 601) in the control system 700 (or 1400) so far may also be displayed on the information presentation monitor 1902 in addition to the determination basis.

The doctor looks at the operative field image displayed on the main surgery video display monitor 1901 and the determination basis displayed on the information presentation monitor 1902 (step S2001), and checks whether or not the operation of the medical robot device 120 predicted by the control system 700 (or 1400) using the neural network model is different from his/her own determination (step S2002).

Here, in a case where the doctor can confirm that the determination basis presented on the information presentation monitor 1902 is not different from his/her own determination (Yes in step S2002), the operation of the medical robot device 120 based on the target command-related information output by the motion predictor 502 (or the E2E predictor 601) meets the intention of the doctor. Therefore, without receiving a correction instruction from a doctor. On the basis of the target command-related information output from the motion predictor 502 (or the E2E predictor 601), the operation of the arm of the medical robot device 120 is controlled as it is (step S2004).

Meanwhile, in a case where the doctor confirms that the determination basis presented on the information presentation monitor 1902 is different from his/her own determination (No in step S2002), the doctor corrects the determination basis displayed on information presentation monitor 1902 using the input device 144 (step S2003). For example, the doctor manually instructs (for example, via UI) to change the position of the heat map of the instrument, environment, and gaze point on which the image is recognized for the heat map image displayed on the information presentation monitor 1902 as the determination basis.

Note that the doctor can instruct correction of the determination basis by, for example, a touch operation on the screen of the information presentation monitor 1902 or voice using the input device 144. Furthermore, the doctor may directly correct the operation of the arm of the medical robot device 120 using the master device of the medical robot device 120.

When the doctor issues a correction instruction of the determination basis, the motion predictor 502 (or the E2E predictor 601) corrects and outputs the target command-related information on the basis of the determination basis corrected and instructed by the doctor, and controls the operation of the arm of the medical robot device 120 (step S2004).

Note that, in a case where a correction instruction of the determination basis is issued by the doctor, the control system 700 (or 1400) may perform reinforcement learning of the learner (image recognizer 501 and motion predictor 502, or E2E predictor 602) according to the correction instruction from the doctor. Alternatively, the control system 700 (or 1400) may correct the target command on a rule basis in response to a correction instruction from the doctor.

When the arm of the medical robot device 120 is operated, the line-of-sight direction and the field of view of the endoscope 110 supported at the distal end thereof are changed, and the position where the operative field image is electronically cut out from the captured image of the endoscope 110 is moved. Then, the operative field image after the angle of view is moved is displayed on the main surgery video display monitor 1901 (step S2005) .

The doctor observes the new operative field image displayed on the main surgery video display monitor 1901. Furthermore, the control system 700 (or 1400) repeatedly executes the motion prediction of the arm of the medical robot device 120 and the presentation of the determination basis of the prediction to the information presentation monitor 1902 on the basis of the captured image of the endoscope 110 after the movement and the motion information and the sensor information output from the medical robot device 120.

According to the operation procedure illustrated in Fig. 20, the doctor can confirm that the operation of the medical robot device 120 is not different from his/her own determination, and can smoothly perform the surgery while manually instructing correction when the operation is different from his/her own determination.

G. Relearning of Learner

In section F described above, it has been described that, in a case where the determination basis of the learner (image recognizer 501 and motion predictor 502 or E2E predictor 601) used by the control system 700 (or 1400) is different from the doctor's determination, it is necessary to perform reinforcement learning of the learner and correction of the target command on a rule basis.

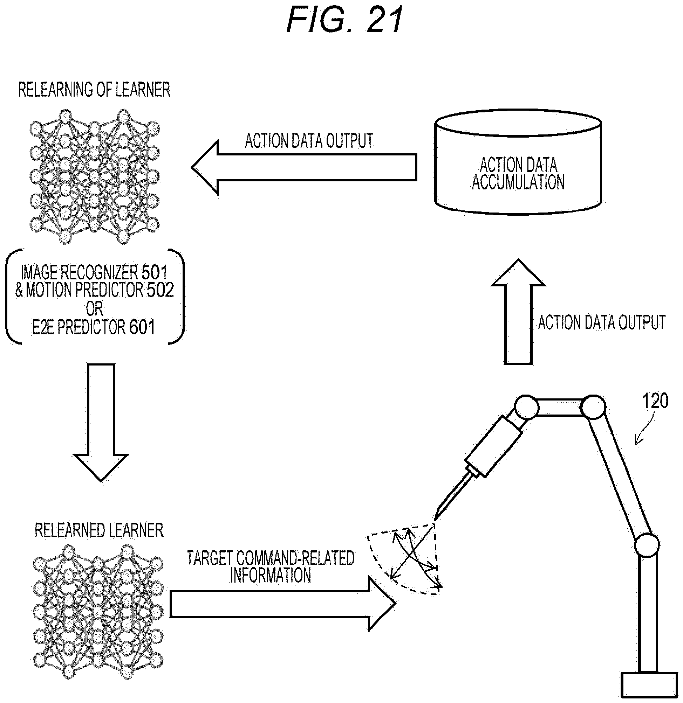

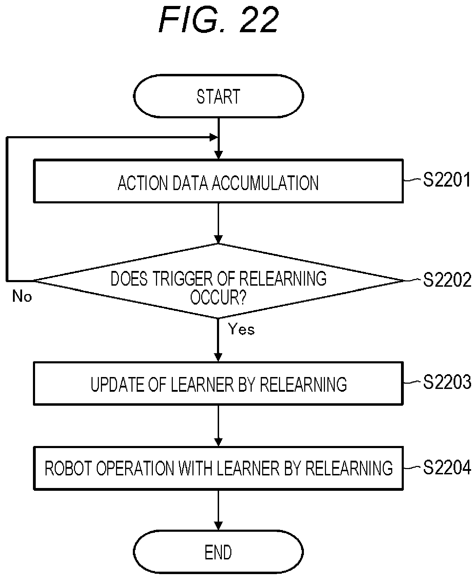

In this section, processing of performing relearning so that the determination basis of the learner does not differ from the determination of the doctor (or the difference between the determination basis of the learner and the determination of the doctor is reduced or becomes zero) will be described. Fig. 21 schematically illustrates a procedure in a case where relearning of the learner is performed in the control system 700 (or 1400). Furthermore, Fig. 22 illustrates an operation procedure for performing relearning of the learner in the control system 700 (or 1400) in the form of a flowchart.

As illustrated in Fig. 19, when a doctor performs an endoscopic surgery using the endoscopic surgery system 100 to which the present disclosure is applied, operation data of the medical robot device 120 is sequentially accumulated (step S2201).

The operation data here includes a combination of input data to the learner, output data, and doctor's determination. Specifically, the input data to the learner includes a captured image of the endoscope 110, motion information of the robot, and sensor information of the robot. Furthermore, the output data from the learner is target command-related information of the medical robot device 120 predicted by the learner. Furthermore, the doctor's determination includes information regarding a doctor's instruction (presence or absence of correction instruction and contents of correction of determination basis) for presentation of the determination basis of the learner.

Here, when a trigger for relearning of the learner occurs (Yes in step S2202), the learner is updated by relearning using the accumulated operation data (step S2203).

Note that the relearning trigger is an arbitrary event. For example, when the accumulated operation data reaches a certain amount or when an operator such as a doctor instructs relearning may be used as a trigger of relearning.

Then, the control system 700 (or 1400) is operated by the learner updated by relearning (step S2204), and the operation of the endoscopic surgery system 100 is continued.

According to the operation procedure illustrated in Fig. 22, it is possible to perform relearning of the learner so as to implement the operation of the medical robot device 120 that is not different from the determination of the doctor in the process in which the doctor performs the surgery while confirming the difference between the operation of the medical robot device 120 and his own determination.

H. Autonomous Learning of Learner

In section F described above, it has been described that, in a case where the determination basis of the learner (image recognizer 501 and motion predictor 502 or E2E predictor 601) used by the control system 700 (or 1400) is different from the doctor's determination, it is necessary to perform reinforcement learning of the learner and correction of the target command on a rule basis.

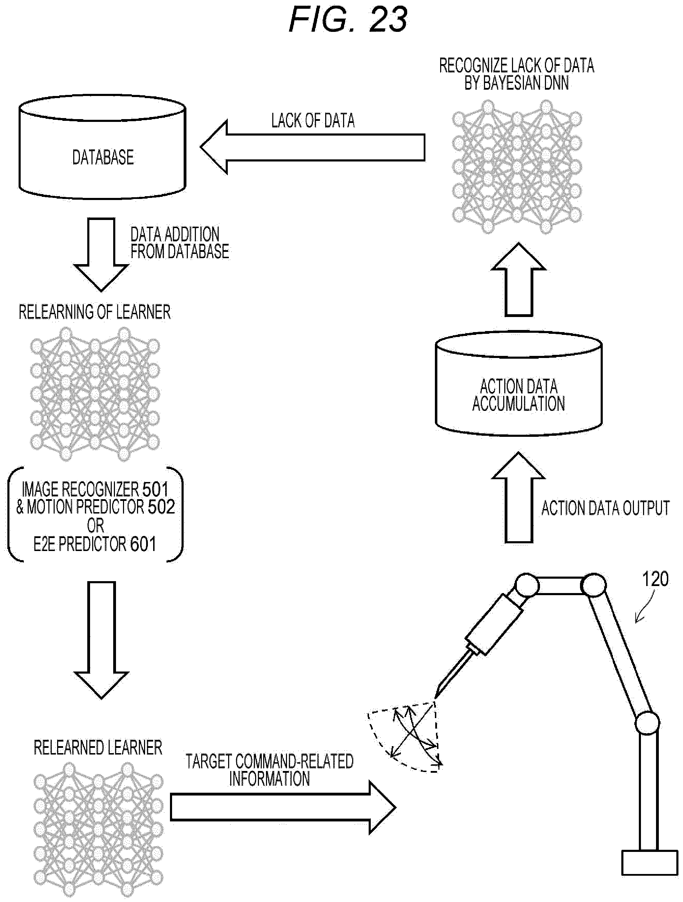

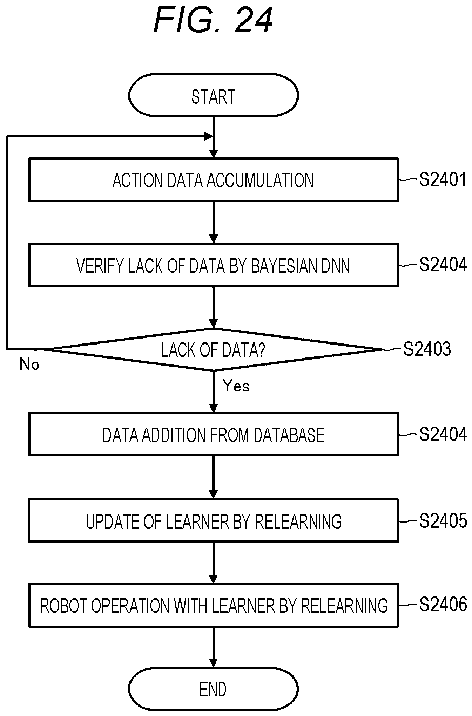

In this section, a process of performing autonomous learning so that the determination basis of the learner does not differ from the determination of the doctor will be described. Fig. 23 schematically illustrates a procedure in a case where autonomous learning of the learner is performed in the control system 700 (or 1400). Furthermore, Fig. 24 illustrates an operation procedure for performing autonomous learning of the learner in the control system 700 (or 1400) in the form of a flowchart.

As illustrated in Fig. 19, when a doctor performs endoscopic surgery using the endoscopic surgery system 100 to which the present disclosure is applied, operation data or manipulation data of the medical robot device 120 is sequentially accumulated (step S2401).

The operation data here includes a combination of input data to the learner, output data, and doctor's determination. Specifically, the input data to the learner includes a captured image of the endoscope 110, motion information of the robot, and sensor information of the robot. Furthermore, the output data from the learner is target command-related information of the medical robot device 120 predicted by the learner. Furthermore, the doctor's determination includes information regarding a doctor's instruction (presence or absence of correction instruction and contents of correction of determination basis) for presentation of the determination basis of the learner.

Then, whether the data of the learner is insufficient is verified by the Bayesian DNN (step S2402). Here, in a case where the lack of data of the learner is recognized by the Bayesian DNN (Yes in step S2403), data is added from the database to compensate for the lack of data (step S2404), relearning of the learner is performed, and the learner is updated (step S2405). That is, in this operation procedure, the estimation result by the Bayesian DNN serves as a trigger for relearning. The database may be an external database.

Then, the control system 700 (or 1400) is operated by the learner updated by relearning (step S2406), and the operation of the endoscopic surgery system 100 is continued.

According to the operation procedure illustrated in Fig. 24, it is possible to perform autonomous learning of the learner so as to implement the operation of the medical robot device 120 that is not different from the determination of the doctor in the process in which the doctor performs the surgery while confirming the difference between the operation of the medical robot device 120 and his own determination. Furthermore, the medical robot device 120 may learn motion prediction or operation or manipulation prediction by reinforcement learning.

INDUSTRIAL APPLICABILITY

The present disclosure has been described in detail above with reference to specific embodiments. However, it is obvious that those skilled in the art can make modifications and substitutions of the embodiments without departing from the gist of the present disclosure.

In the present specification, the embodiments in which the present disclosure is applied to a medical robot device that supports an endoscope has been mainly described, but the gist of the present disclosure is not limited thereto. The present disclosure can be similarly applied to a medical robot device that supports a medical instrument other than an endoscope, for example, forceps, a pneumoperitoneum tube, an energy treatment tool, tweezers, a retractor, or the like at the distal end, and further a robot device that performs information presentation, operation instruction, or the like without using a support tool or the like, to present a determination basis, uncertainty, or reliability of an estimation result by deep learning.

In short, the present disclosure has been described in the form of exemplification, and the contents described in the present specification should not be interpreted in a limited manner. In order to determine the gist of the present disclosure, the claims should be taken into consideration.

Note that the present disclosure can have the following configurations.

(1) A medical support system including:

a control unit;
a recognition unit that recognizes an operative field environment; and
a machine learning model that estimates an operation performed by the medical support system on the basis of a recognition result of the recognition unit,
in which the control unit outputs determination basis information regarding the operation estimated by the machine learning model to an information presentation unit.

(2) The medical support system according to (1),
in which the control unit further includes a calculation unit that calculates reliability regarding an estimation result of the machine learning model, and outputs the reliability to the information presentation unit.
(3) The medical support system according to any one of (1) and (2),

in which the machine learning model estimates a target command for an arm supporting a medical instrument, and the control unit outputs determination basis information regarding the target command estimated by the machine learning model to an information presentation unit.

(4) The medical support system according to (2),
in which the calculation unit calculates the reliability using Bayesian deep learning.
(5) The medical support system according to (3),
in which the control unit outputs information regarding a gaze region observed at a time of estimating the target command and/or a recognized target portion.
(6) The medical support system according to any one of (1) to (5),

in which the medical support system further records response data of a user to the determination basis information output to the information presentation unit, and
the control unit performs relearning of the machine learning model on the basis of the response data of the user.

(7) The medical support system according to (5),
in which the control unit outputs a heat map image indicating the gaze region observed at the time of estimating the target command and/or the recognized target portion.
(8) The medical support system according to (7),

in which the medical instrument is an endoscope, and
the control unit outputs the heat map image having a wider angle of view than a monitor image of the endoscope.

(9) The medical support system according to any one of (7) and (8),
in which the control unit outputs the heat map image generated on the basis of a Grad-Cam algorithm.
(10) The medical support system according to any one of (2) and (4),
in which the control unit calculates, as the reliability, a numerical value indicating at least one of lack of data, an unknown environment or condition, or variance or accuracy of a prediction result when the machine learning model estimates the operation of the medical support system.
(11) The medical support system according to any one of (2) and (4),
in which the control unit calculates reliability of a plurality of candidates for the operation of the medical support system estimated by the machine learning model.
(12) The medical support system according to (3),

in which the medical instrument is an endoscope, and
the information presentation unit presents the determination basis information in a screen displaying an operative field image captured by the endoscope.

(13) The medical support system according to any one of (1) to (12), further including

an input unit that receives an instruction from a user for the determination basis information presented by the information presentation unit,
in which the control unit performs control so that the machine learning model estimates the operation of the medical support system on the basis of the determination basis information corrected via the input unit.

(14) The medical robot device according to (2),
in which the medical robot device performs autonomous learning of the machine learning model using operation data including the operation performed by the medical support system, the determination basis information regarding the operation estimated by the machine learning model, and a user's instruction for the determination basis information on the basis of the reliability calculated by the calculation unit.
(15) A medical support method in a medical support system, the medical support method including:

a recognition step of recognizing an operative field environment;
an estimation step of estimating, by a machine learning model, an operation performed by the medical support system on the basis of a recognition result in the recognition step; and
a step of outputting determination basis information regarding the operation estimated by the machine learning model to an information presentation unit.

(16) A computer program described in a computer readable format to execute processing of medical support in a medical support system on a computer, the computer program causing the computer to function as:

a recognition unit that recognizes an operative field environment;
an estimation unit that estimates, by a machine learning model, an operation performed by the medical support system on the basis of a recognition result in the recognition step; and
an output unit that outputs determination basis information regarding the operation estimated by the machine learning model to an information presentation unit.

REFERENCE SIGNS LIST

100     Endoscope system
101     Surgeon

| | |
|---|---|
| 102 | Surgical bed |
| 103 | Patient |
| 110 | Endoscope |
| 111 | Lens barrel |
| 112 | camera head |
| 120 | Medical robot device |
| 130 | Medical instrument group |
| 131, 132 | Medical instrument |
| 140 | Cart |
| 141 | CCU |
| 142 | Light source device |
| 143 | Robot arm control device |
| 144 | Input device |
| 145 | Treatment tool control device |
| 146 | Air film device |
| 147 | Recorder |
| 148 | Printer |
| 149 | Display device |
| 151, 152 | Trocar |
| 301 | Lens unit |
| 302 | Imaging unit |
| 303 | Drive unit |
| 304 | Communication unit |
| 305 | Camera head control unit |
| 311 | Communication unit |
| 310 | Passive joint unit |
| 311 | Encoder |
| 320 | Active joint unit |
| 321 | Actuator |
| 322 | Torque sensor |
| 323 | Encoder |
| 330 | Passive slide mechanism |
| 331 | Sensor |
| 340 | Sensor unit |
| 410 | Active joint unit |
| 411 | Actuator |
| 412 | Torque sensor |
| 413 | Encoder |
| 420 | Passive joint unit |
| 421 | Encoder |
| 501 | Image recognizer |
| 502 | Motion predictor |
| 601 | E2E predictor |
| 700 | Control system |
| 701 | Attention information presentation unit |
| 702 | Reliability presentation unit |
| 1400 | Control system |
| 1401 | Attention information presentation unit |
| 1402 | Reliability presentation unit |

**Claims**

1.  A medical support system comprising:

    a control unit;
    a recognition unit that recognizes an operative field environment; and
    a machine learning model that estimates an operation performed by the medical support system on a basis of a recognition result of the recognition unit,
wherein the control unit outputs determination basis information regarding the operation estimated by the machine learning model to an information presentation unit.

2. The medical support system according to claim 1,
wherein the control unit further includes a calculation unit that calculates reliability regarding an estimation result of the machine learning model, and outputs the reliability to the information presentation unit.

3. The medical support system according to claim 1,

wherein the machine learning model estimates a target command for an arm supporting a medical instrument, and the control unit outputs determination basis information regarding the target command estimated by the machine learning model to an information presentation unit.

4. The medical support system according to claim 2,
wherein the calculation unit calculates the reliability using Bayesian deep learning.

5. The medical support system according to claim 3,
wherein the control unit outputs information regarding a gaze region observed at a time of estimating the target command and/or a recognized target portion.

6. The medical support system according to claim 1,

wherein the medical support system further records response data of a user to the determination basis information output to the information presentation unit, and
the control unit performs relearning of the machine learning model on a basis of the response data of the user.

7. The medical support system according to claim 5,
wherein the control unit outputs a heat map image indicating the gaze region observed at the time of estimating the target command and/or the recognized target portion.

8. The medical support system according to claim 7,
wherein the medical instrument is an endoscope, and the control unit outputs the heat map image having a wider angle of view than a monitor image of the endoscope.

9. The medical support system according to claim 7,
wherein the control unit outputs the heat map image generated on a basis of a Grad-Cam algorithm.

10. The medical support system according to claim 2,
wherein the control unit calculates, as the reliability, a numerical value indicating at least one of lack of data, an unknown environment or condition, or variance or accuracy of a prediction result when the machine learning model estimates the operation of the medical support system.

11. The medical support system according to claim 2,
wherein the control unit calculates reliability of a plurality of candidates for the operation of the medical support system estimated by the machine learning model.

12. The medical support system according to claim 3,
wherein the medical instrument is an endoscope, and the information presentation unit presents the determination basis information in a screen displaying an operative field image captured by the endoscope.

13. The medical support system according to claim 1, further comprising

an input unit that receives an instruction from a user for the determination basis information presented by the information presentation unit,
wherein the control unit performs control so that the machine learning model estimates the operation of the medical support system on a basis of the determination basis information corrected via the input unit.

**14.** The medical robot device according to claim 2,
wherein the medical robot device performs autonomous learning of the machine learning model using operation data including the operation performed by the medical support system, the determination basis information regarding the operation estimated by the machine learning model, and a user's instruction for the determination basis information on a basis of the reliability calculated by the calculation unit.

**15.** A medical support method in a medical support system, the medical support method comprising:

a recognition step of recognizing an operative field environment;
an estimation step of estimating, by a machine learning model, an operation performed by the medical support system on a basis of a recognition result in the recognition step; and
a step of outputting determination basis information regarding the operation estimated by the machine learning model to an information presentation unit.

**16.** A computer program described in a computer readable format to execute processing of medical support in a medical support system on a computer, the computer program causing the computer to function as:

a recognition unit that recognizes an operative field environment;
an estimation unit that estimates, by a machine learning model, an operation performed by the medical support system on a basis of a recognition result in the recognition step; and
an output unit that outputs determination basis information regarding the operation estimated by the machine learning model to an information presentation unit.

EP 4 191 606 A1

## FIG. 1

# FIG. 2

DISPLAY DEVICE
149

CAPTURED IMAGE

CAMERA HEAD
112

CONTROL SIGNAL TO
CAMERA HEAD 112

CCU
141

RECOGNITION
INFORMATION

ROBOT ARM
CONTROL DEVICE
143

MEDICAL ROBOT
DEVICE
120

CONTROL SYSTEM 200

INPUT DEVICE
144

MOTION INFORMATION
SENSOR INFORMATION

EP 4 191 606 A1

# FIG. 3

```
┌─────────────────────────────────────────────────────────────────────┐
│                                                                       │
│   ┌──────────────┐      ┌──────────────┐      ┌──────────────┐        │
│   │  LENS UNIT   │─────▶│ IMAGING UNIT │─────▶│ COMMUNICATION│        │
│   │     301      │      │     302      │      │     UNIT     │        │
│   │              │      │              │◀─────│     304      │        │
│   └──────────────┘      └──────────────┘      └──────────────┘        │
│          ▲                                            ▲               │
│          │              ┌──────────────┐      ┌──────────────┐        │
│          │              │  DRIVE UNIT  │      │ CAMERA HEAD  │        │
│          └──────────────│     303      │      │ CONTROL UNIT │        │
│                         │              │◀─────│     305      │        │
│                         └──────────────┘      └──────────────┘        │
│   CAMERA HEAD 112                                                     │
└─────────────────────────────────────────────────────────────────────┘
                                  │
                                  └── 311
                    ┌─────────────────────────────┐
                    │                             │
                    │            CCU              │
                    │            141              │
                    │                             │
                    └─────────────────────────────┘
                                  │
                    ┌─────────────────────────────┐
                    │                             │
                    │   ROBOT ARM CONTROL DEVICE  │
                    │            143              │
                    │                             │
                    └─────────────────────────────┘
```

# FIG. 4

INPUT DEVICE
144

CONTROL DEVICE
143

MEDICAL ROBOT DEVICE 120

ACTIVE JOINT UNIT 410

ACTUATOR
411

TORQUE SENSOR
412

ENCODER
413

PASSIVE JOINT UNIT 420

ENCODER
421

SENSOR UNIT
(IMU OR THE LIKE)
430

## FIG. 5

CAPTURED IMAGE OF SURGICAL SITE BY ENDOSCOPE

MOTION INFORMATION OF ROBOT ARM

DISTAL END POSITION/SPEED/ ACCELERATION
EACH JOINT POSTURE

SENSOR INFORMATION OF ROBOT ARM

IMU
EACH JOINT TORQUE
DISTAL END PORTION FORCE SENSOR

IMAGE RECOGNIZER 501 (WITHIN CCU 141)

NN MODEL LEARNED BY DL

INSTRUMENT RECOGNITION INFORMATION

POSITION/POSTURE
OPEN/CLOSED AND OPERATION STATE
TYPE

ENVIRONMENT RECOGNITION INFORMATION

DEPTH/ENVIRONMENT MAP
ORGAN/INSTRUMENT/MATERIAL

MOTION PREDICTOR 502 (WITHIN CONTROL DEVICE 143)

NN MODEL LEARNED BY DL

TARGET COMMAND-RELATED INFORMATION

CAMERA TARGET POSITION/POSTURE/ SPEED/ACCELERATION
INSTRUMENT TARGET POSITION/POSTURE/ SPEED/ACCELERATION/OPERATION FORCE
MIDDLE VIEW POINT/LINE-OF-SIGHT VECTOR (TARGET OBJECT POSITION/DISTANCE/ VECTOR POSTURE)
ELECTRONIC CUT-OUT POSITION/DISTANCE

CONTROL SYSTEM 200

# FIG. 6

CAPTURED IMAGE OF
SURGICAL SITE BY ENDOSCOPE

MOTION INFORMATION
OF ROBOT ARM

DISTAL END POSITION/SPEED/
ACCELERATION
EACH JOINT POSTURE

SENSOR INFORMATION
OF ROBOT ARM

IMU
EACH JOINT TORQUE
DISTAL END PORTION FORCE SENSOR

E2E PREDICTOR 601

NN MODEL LEARNED BY DL

TARGET COMMAND-RELATED
INFORMATION

CAMERA TARGET POSITION/POSTURE/
SPEED/ACCELERATION
INSTRUMENT TARGET POSITION/POSTURE/
SPEED/ACCELERATION/OPERATION FORCE
MIDDLE VIEW POINT/LINE-OF-SIGHT VECTOR
(TARGET OBJECT POSITION/DISTANCE/
VECTOR POSTURE)
ELECTRONIC CUT-OUT POSITION/DISTANCE

120

CONTROL SYSTEM 200

# FIG. 7

## FIG. 8

## FIG. 9

## FIG. 10

## FIG. 11

# FIG. 12

# FIG. 13

START

DATA INPUT —S1301

IMAGE RECOGNITION —S1302

HEAT MAP DISPLAY OF DETERMINATION BASIS —S1303

MOTION PREDICTION —S1304

RELIABILITY PRESENTATION OF DATA —S1305

ROBOT OPERATION —S1306

END

# FIG. 14

CONTROL SYSTEM 1400

CAPTURED IMAGE OF SURGICAL SITE BY ENDOSCOPE

MOTION INFORMATION OF ROBOT ARM

SENSOR INFORMATION OF ROBOT ARM

E2E PREDICTOR 601

TARGET COMMAND-RELATED INFORMATION

120

ATTENTION INFORMATION PRESENTATION UNIT 1401

RELIABILITY PRESENTATION UNIT 1402

HEAT MAP DISPLAY

EXPLANATION DATA PRESENTATION

EXPLANATION DATA
- LACK OF DATA
- UNKNOWN ENVIRONMENT / CONDITION
- VARIANCE AND ACCURACY OF PREDICTION RESULT

EP 4 191 606 A1

# FIG. 15

```
          ┌─────────────┐
          │    START    │
          └─────────────┘
                 │
                 ▼
     ┌──────────────────────┐
     │     DATA INPUT       │──── S1501
     └──────────────────────┘
                 │
                 ▼
     ┌──────────────────────┐
     │ MOTION PREDICTION BY E2E │──── S1502
     └──────────────────────┘
                 │
                 ▼
     ┌──────────────────────────────────┐
     │ HEAT MAP DISPLAY OF DETERMINATION BASIS │──── S1503
     └──────────────────────────────────┘
                 │
                 ▼
     ┌──────────────────────────┐
     │ RELIABILITY PRESENTATION OF DATA │──── S1504
     └──────────────────────────┘
                 │
                 ▼
     ┌──────────────────────┐
     │    ROBOT OPERATION    │──── S1505
     └──────────────────────┘
                 │
                 ▼
          ┌─────────────┐
          │     END     │
          └─────────────┘
```

FIG. 16

EP 4 191 606 A1

## FIG. 17

1611

## FIG. 18

1612

## FIG. 19

MAIN SURGERY VIDEO DISPLAY MONITOR 1901

OPERATIVE FIELD IMAGE

OPERATIVE FIELD IMAGE

120

DOCTOR

DETERMINATION BASIS

CORRECTION INSTRUCTION

GAZE POINT

Grasper

Scissors

TARGET COMMAND

INFORMATION PRESENTATION MONITOR 1902

EP 4 191 606 A1

# FIG. 20

```
        ┌──────────────┐
        │    START     │
        └──────┬───────┘
               │
               ▼
   ┌──────────────────────────────┐
   │ DETERMINATION BASIS PRESENTATION │──S2001
   └──────────────┬───────────────┘
                  │
Yes               ▼
┌─────◇ IS DETERMINATION OF DOCTOR ◇──S2002
│         SAME?
│                 │ No
│                 ▼
│  ┌──────────────────────────────┐
│  │ CORRECTION INSTRUCTION BY DOCTOR │──S2003
│  └──────────────┬───────────────┘
│                 │
└─────────────────┤
                  ▼
   ┌──────────────────────────────┐
   │        ROBOT OPERATION        │──S2004
   └──────────────┬───────────────┘
                  │
                  ▼
   ┌──────────────────────────────┐
   │   OPERATIVE FIELD IMAGE OUTPUT │──S2005
   │    AFTER ROBOT OPERATION       │
   └──────────────┬───────────────┘
                  │
                  ▼
        ┌──────────────┐
        │     END      │
        └──────────────┘
```

# FIG. 21

RELEARNING OF LEARNER

ACTION DATA OUTPUT

ACTION DATA
ACCUMULATION

IMAGE RECOGNIZER 501
& MOTION PREDICTOR 502
OR
E2E PREDICTOR 601

ACTION DATA OUTPUT

120

RELEARNED LEARNER

TARGET COMMAND-RELATED
INFORMATION

# FIG. 22

```
              ┌──────────────┐
              │    START     │
              └──────┬───────┘
                     │
       ┌─────────────▼──────────────────────┐
   ┌──▶│     ACTION DATA ACCUMULATION        │─── S2201
   │   └─────────────┬──────────────────────┘
   │                 │
   │          ┌──────▼──────┐
   │         ╱               ╲
   │  No   ╱ DOES TRIGGER OF   ╲  S2202
   └──────╱  RELEARNING OCCUR?  ╲
          ╲                     ╱
           ╲                   ╱
            ╲──────┬──────────╱
                   │ Yes
       ┌───────────▼──────────────────────┐
       │  UPDATE OF LEARNER BY RELEARNING  │─── S2203
       └───────────┬──────────────────────┘
                   │
       ┌───────────▼──────────────────────────────┐
       │ ROBOT OPERATION WITH LEARNER BY RELEARNING│─── S2204
       └───────────┬──────────────────────────────┘
                   │
              ┌────▼────┐
              │   END   │
              └─────────┘
```

# FIG. 23

RECOGNIZE LACK OF DATA
BY BAYESIAN DNN

LACK OF DATA

DATABASE

DATA ADDITION
FROM DATABASE

RELEARNING OF LEARNER

IMAGE RECOGNIZER 501
& MOTION PREDICTOR 502
OR
E2E PREDICTOR 601

ACTION DATA
ACCUMULATION

ACTION DATA OUTPUT

120

RELEARNED LEARNER

TARGET COMMAND-RELATED
INFORMATION

# FIG. 24

```
            ┌─────────────┐
            │    START    │
            └─────────────┘
                   │
   ┌──────────────▼──────────────────────┐
   │   ACTION DATA ACCUMULATION           │──── S2401
   └──────────────┬──────────────────────┘
                  │
   ┌──────────────▼──────────────────────┐
   │  VERIFY LACK OF DATA BY BAYESIAN DNN │──── S2404
   └──────────────┬──────────────────────┘
                  │
              ╱───▼────╲
   No   ◀────╱ LACK OF   ╲──── S2403
            ╲  DATA?     ╱
              ╲────┬───╱
                   │ Yes
   ┌──────────────▼──────────────────────┐
   │   DATA ADDITION FROM DATABASE        │──── S2404
   └──────────────┬──────────────────────┘
                  │
   ┌──────────────▼──────────────────────┐
   │   UPDATE OF LEARNER BY RELEARNING    │──── S2405
   └──────────────┬──────────────────────┘
                  │
   ┌──────────────▼──────────────────────┐
   │ ROBOT OPERATION WITH LEARNER BY      │──── S2406
   │ RELEARNING                           │
   └──────────────┬──────────────────────┘
                  │
            ┌─────▼───────┐
            │    END      │
            └─────────────┘
```

## INTERNATIONAL SEARCH REPORT

| International application No. |
| --- |
| PCT/JP2021/022041 |

### A. CLASSIFICATION OF SUBJECT MATTER
Int.Cl. G16H40/60(2018.01)i, A61B34/00(2016.01)i
FI: G16H40/60, A61B34/00

According to International Patent Classification (IPC) or to both national classification and IPC

### B. FIELDS SEARCHED
Minimum documentation searched (classification system followed by classification symbols)
Int.Cl. G16H40/60, A61B34/00

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | US 2018/0296281 A1 (BIO-MEDICAL ENGINEERING (HK) LIMITED) 18 October 2018 (2018-10-18), paragraphs [0158], [0167], [0172], [0188]-[0200], [0205]-[0207], [0210], [0211] | 1-7, 9, 10, 12, 15, 16 |
| A | | 8, 11, 13, 14 |

☐ Further documents are listed in the continuation of Box C.   ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 18 August 2021 | 31 August 2021 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

INTERNATIONAL SEARCH REPORT
Information on patent family members

International application No.

PCT/JP2021/022041

US 2018/0296281 A1 18 October 2018     WO 2018/188466 A1
                                      CN 108685560 A

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020027507 A

**Non-patent literature cited in the description**

- **ALEX KENDALL ; YARIN GAL.** What Uncertainties Do We Need in Bayesian Deep Learning for Computer Vison. *NIPS,* 2017

- **YARIN GAL ; ZOUBIN GHAHRAMANI.** Dropout as Bayesian Approximation: Representing Model Uncertainty in Deep Learning. *ICML,* 2016